# EUROPÄISCHE PATENTSCHRIFT

(11) **EP 1 720 978 B1**
(45) Veröffentlichungstag und Bekanntmachung des Hinweises auf die Patenterteilung: **14.11.2007**
(21) Anmeldenummer: 05715545.9
(22) Anmeldetag: 25.02.2005
(51) Int. Cl.: C12N 5/00, C12M 3/00

(54) **MAGNETISCHE MANIPULATION VON BIOLOGISCHEN PROBEN**
MAGNETIC MANIPULATION OF BIOLOGICAL SAMPLES
MANIPULATION MAGNETIQUE D'ECHANTILLONS BIOLOGIQUES

(30) Priorität: 01.03.2004 DE 102004009985
(43) Veröffentlichungstag der Anmeldung: 15.11.2006
(73) Patentinhaber: Fraunhofer-Gesellschaft zur Förderung der angewandten Forschung e.V., 80686 München (DE)
(72) Erfinder: FUHR, Günter, 13187 Berlin (DE)
(74) Vertreter: Hertz, Oliver
(86) Internationale Anmeldenummer: PCT/EP2005/002004
(87) Internationale Veröffentlichungsnummer: WO 2005/083057

(56) Entgegenhaltungen:
- WO-A-03/043931
- US-B1- 6 355 491
- US-B1- 6 368 838
- DATABASE WPI Section Ch, Week 200142 Derwent Publications Ltd., London, GB; Class D16, AN 2001-390726 XP002312716 & CN 1 289 842 A (INST CHEM METALLURGY CHINESE ACAD SCI) 4. April 2001 (2001-04-04)

## Beschreibung

Die Erfindung betrifft Zellträger mit den Merkmalen des Oberbegriffs von Anspruch 1, Kulturträger, die eine Vielzahl derartiger Zellträger umfassen, Manipulationseinrichtungen für die Verwendung derartiger Zellträger zur Manipulation biologischer Proben, und Verfahren zur Manipulation von biologischen Proben, wie zum Beispiel von Zellen und/oder Zellbestandteilen.

Wichtige Aufgaben der Biotechnologie und Medizin bestehen in der möglichst automatisierten Erzeugung und Prozessierung von Zellgruppen und Zellverbänden aus einzelnen biologischen Zellen. Es sollen zum Beispiel Zellen mit gewünschten Eigenschaften gruppiert, behandelt, vermehrt oder allgemein örtliche und/oder zeitlich manipuliert werden. Eine wesentliche Voraussetzung für Zellkultivierungen bildet der Kontakt der biologischen Zellen zu einer festen Oberfläche (adhärente Zellen). Viele zum Beispiel für Zelltherapien oder in der Biotechnologie relevante Zelltypen (z. B. Fibroblasten, Makrophagen, Lymphozyten, Stammzellen) können ohne diese Berührung nicht überleben oder zumindest sich nicht teilen. Daher gewinnen molekular maskierte oder mikro- und nanostrukturierte Oberflächen an Bedeutung.

In der Praxis haben sich insbesondere wegen der gewünschten Sterilität und geringen Kosten miniaturisierte Systeme in Form von so genannten "Bio-Lab's on Chip" als vorteilhaft erwiesen. Diese werden oft als fluidisches Mikrokanalsystem mit zum Beispiel optischen oder elektrischen Funktionskomponenten zur Separierung, Behandlung, Gruppierung und Charakterisierung von biologischen Zellen hergestellt. Nachteilig an den herkömmlichen Mikrokanalsystemen ist jedoch, dass in diesen die Zellen nur im suspendierten, relativ zu festen Oberflächen ungebunden Zustand zum Beispiel durch passive Strömungen, mit elektrischen Feldern (Elektroosmose, Dielektrophorese, Elektrophorese) oder durch optische Kräfte bewegt werden können. Die o. g. Kontakte mit festen Oberflächen sind dadurch gerade ausgeschlossen. Haften die Zellen dennoch an Oberflächen an, so ist ein Ablösen in der Regel schwierig und nur auf biochemischem Weg (Trypsinieren) oder mit starkem mechanischen Stress und Zellverlusten möglich.

Mit den herkömmlichen Mikrokanalsystemen sind daher Zellbehandlungen mit Oberflächen- oder Zellkontakten oder Kultivierungen von Zellen nur beschränkt durchführbar. Zur Manipulation von adhärenten Zellen stehen derzeit keine praktikablen Hilfsmittel zur Verfügung. Adhärente Zellen lassen sich einzeln weder relativ zum Kanalsystem noch relativ zueinander bewegen. Da aber gerade die Adhärenz der Zellen von grundlegender Bedeutung ist, besteht hier ein starker Bedarf an einer Technik, die im Forschungslabor und in der Industrie insbesondere bei der Umsetzung medizinischer Zelltherapien verwendbar ist.

Es ist bekannt, biologische Zellen in Suspension mit magnetischen Teilchen (so genannte Nano- oder Mikrobeads) zu koppeln, um dann mit einem externen Magnetfeld eine Zellbewegung auszuführen. Die Anwendung magnetischer Beads, die eine sphärische Geometrie besitzen, ist bisher auf die unspezifische Manipulation von Zellgruppen (zum Beispiel bei der Sammlung von suspendierten Zellen) beschränkt. Eine in der Lage definierte oder individuelle Handhabung Bead-gekoppelter Zellen ist bislang nicht möglich. Bead-Zell-Komplexe zeigen starke Wechselwirkungen, so dass es schnell zu unerwünschten Aggregationen (Partikel-Partikel-Wechselwirkungen über das Magnetfeld oder die Oberflächen) kommen kann. Des weiteren ist eine individuelle Entnahme von Bead-Zell-Komplexen aus einer Suspension ein bislang ungelöstes Problem.

US-B-6355491 offenbart einen Träger zur Aufnahme einer biologischen Probe mit einem Bodenelement, einem Magnetelement und einem adhäsionsfördernd gebildeten Aufnahmeelement. Der herkömmliche Träger ist auf einem Basisteil unbeweglich fixiert. Er ist für die Aufnahme von Biomolekülen in Flüssigkeiten, nicht jedoch zur Aufnahme biologischer Zellen geeignet. Aus CN-A-12898842 ist ein Träger für biologische Zellen mit einem Magnetelement und einem adhäsionsfördernd gebildeten Aufnahmeelement bekannt, der allerdings keine Beweglichkeit auf einer Basisfläche ermöglicht. Magnetische Partikel für biotechnologische Anwendungen werden auch in WO 03/043931 A1 beschrieben.

Die Aufgabe der Erfindung ist es, verbesserte Techniken zur Manipulation biologischer Proben, wie z.B. von Zellen, Zellgruppen, Zellbestandteilen oder biologisch wirksamen Substanzen bereitzustellen, mit denen die Nachteile und Beschränkungen der herkömmlichen fluidischen Mikrosysteme überwunden werden können. Es soll insbesondere eine Möglichkeit geschaffen werden, biologische Proben im adhärenten, an eine Festphase gebundenen Zustand einzeln oder gruppenweise zu bewegen, zu positionieren und/oder definiert in Kontakt zueinander zu bringen, wobei eine möglichst hohe Flexibilität in Bezug auf die Anwendbarkeit von zum Beispiel biochemischen, gentechnischen, medizinischen oder biologischen Verfahrenstechniken erreicht werden soll. Mit der Erfindung soll ferner eine Möglichkeit geschaffen werden, adhärente Zellen zuverlässig einzeln und unter sterilen Bedingungen zu manipulieren.

Diese Aufgabe wird durch einen Zellträger mit den Merkmalen von Patentanspruch 1, einen aus einer Vielzahl derartiger Zellträger zusammengesetzten Kulturträger mit den Merkmalen von Patentanspruch 30, eine Manipulationseinrichtung für biologische Proben mit den Merkmalen von Patentanspruch 36 und Verfahren zur Manipulation biologischer Proben mit den Merkmalen von Patentanspruch 46 gelöst. Vorteilhafte Ausführungsformen und Anwendungen der Erfindung ergeben sich aus den abhängigen Ansprüchen.

Vorrichtungsbezogen basiert die vorliegende Erfindung gemäß einem ersten Aspekt insbesondere auf der technischen Lehre, einen Zellträger zur Aufnahme einer biologischen Probe bereitzustellen, der mindestens ein Magnetelement enthält, das eine Bewegung oder Positionierung des Zellträgers mit einer magnetischen Kraft ermöglicht, wobei ein Bodenelement vorgesehen ist, mit dem der Zellträger mechanisch stabil auf einer festen Oberfläche aufsetzbar und verschiebbar ist. Durch die Schaffung des Bodenelements, das auf einer Unterseite des Zellträgers eine Auflage bildet, wird vorteilhafterweise eine Lagestabilität des Zellträgers sowohl im ruhenden als auch im insbesondere mit der magnetischen Kraft bewegten Zustand erreicht. Die Stabilität des Zellträgers bedeutet, dass dieser auf der festen Oberfläche, die im folgenden als Basisfläche oder Bodenfläche bezeichnet wird, verkippungsfrei angeordnet werden kann. Umorientierungen des Zellträgers relativ zur Basisfläche, wie z.B. eine Kippbewegung, ein Umfallen oder eine komplette Umkehrung um 180°, sind ausgeschlossen. Vorteilhafterweise können erfindungsgemäße Zellträger allein durch die Ausübung der magnetischen Kraft auf der Basisfläche in vorbestimmter Weise verschoben werden. Die magnetische Kraft wird durch eine magnetische Wechselwirkung des Magnetelements, das ein magnetisches und/oder ein magnetisierbares Material enthält, mit einem äußeren Magnetfeld oder einem äußeren magnetisierbaren Material erzeugt.

Vorteilhafterweise werden durch die Fähigkeit des Zellträgers, stabil auf einer festen Basisfläche zu ruhen oder bewegt zu werden, die Nachteile der herkömmlichen magnetischen Mikrobeads überwunden. Es können insbesondere nichtreproduzierbare Aggregationen von Mikrobeads ausgeschlossen werden. Die herkömmliche beadbasierte Manipulation von Zellen bietet zudem nicht die Möglichkeit, planare und komplex strukturierte Oberflächen für den Zellbewuchs in vorgebbarer Größe und Beschaffenheit verfügbar zu machen.

Vorzugsweise umfasst der Zellträger mit dem Magnetelement auf seiner Unterseite das Bodenelement, das die stabile, auf einer festen Basisfläche in mindestens einer Richtung verschiebbare Auflage bildet, und an seiner Oberseite ein Aufnahmeelement, das eine Oberfläche zur Aufnahme der biologischen Probe aufweist. Gemäß einem Merkmal der Erfindung ist das Aufnahmeelement wenigstens in einem vorbestimmten Teilbereich seiner Oberfläche adhäsionsfördernd modifiziert, d.h. es trägt eine Beschichtung und/oder Strukturierung, auf der biologische Zellen bevorzugt anhaften. Für die praktische Anwendung ist es besonders bevorzugt, wenn die adhäsionsfördernde Beschichtung und/oder Strukturierung in einem Teilbereich der Oberfläche und eine adhäsionsbehindernde Beschichtung in einem anderen Teilbereich der Oberfläche vorgesehen sind, wobei die Teilbereiche zusammenhängend oder jeweils unterteilt sein können.

Erfindungsgemäße Zellträger sind vorzugsweise Plättchen, die magnetische Materialien oder magnetisierbare Teile enthalten oder tragen, wobei die Zellträger für die Kraftaufnahme eingerichtet sind und eine Kulturplattform und -oberfläche für Zellen bilden. Die Zellträger sind mit miniaturisierten, außerhalb eines Kulturgefäßes oder Mikrosystems geführten x-y-z-Manipulatoren beweglich. Entsprechend der Größe von Zellen können diese Systeme bis in den Mikrometerbereich miniaturisiert werden und sind in jedem Fall kleiner im Vergleich zu den typischen Abmessungen der Wände des Kulturgefäßes. Die Zellträger können mit Materialien beschichtet sein, die sowohl die Gleitfähigkeit auf den Wänden als auch die Bioverträglichkeit mit den Zellen gewährleisten. Ein breites Spektrum von Materialkombinationen, Material-Anisotropien und stofflichen Gradienten ist realisierbar.

Das Bodenelement des erfindungsgemäßen Zellträgers kann in Abhängigkeit von der konkreten Anwendung und insbesondere der Gestalt der Basisfläche geformt sein, auf der der Zellträger verwendet werden soll. Allgemein kann die Oberflächenform des Bodenelements jede zur Basisfläche komplementäre Form besitzen, wobei das Bodenelement wegen der gewünschten Verschiebbarkeit eine in mindestens einer Richtung krümmungsfreie, gerade Form besitzt.

Gemäß einer bevorzugten Ausführungsform der Erfindung besitzt das Bodenelement eine ebene und strukturfreie Oberflächenstruktur. Vorteilhafterweise wird eine ebene, glatte Auflage gebildet, die insbesondere auf einer ebenen Basisfläche in allen Richtungen parallel zur Basisfläche frei verschiebbar ist. Alternativ kann das Bodenelement eine Oberflächenform besitzen, die eine ebene, strukturierte Auflage bildet. Beispielsweise können drei oder mehr Auflagepunkte vorgesehen sein, die in einer gemeinsamen Ebene liegen und entsprechend ebenfalls eine ebene Auflage des erfindungsgemäßen Zellträgers bilden. In diesem Fall ergeben sich Vorteile für eine verminderte Reibung des Zellträgers gegenüber der Basisfläche. Gemäß einer weiteren Alternative kann das Bodenelement ein bestimmtes Auflageprofil besitzen, das komplementär zur Basisfläche ist, auf welcher der Zellträger verwendet werden soll. Vorteilhafterweise kann durch die Vorgabe des Auflageprofils eine Bewegungsrichtung des Zellträgers auf der Basisfläche nach Art einer Schienenbewegung fest vorgegeben werden. Allgemein kann das Auflageprofil der Oberflächenform des Bodenelements komplett konkav oder konvex oder in Teilbereichen konkav und konvex sein. Wenn das Auflageprofil einen kreisförmigen Querschnitt besitzt, so dass das Bodenelement einen Ausschnitt eines geraden Kreiszylinders bildet, können sich Vorteile für die Anwendung des Zellträgers auf zylinderförmig gekrümmten Basisflächen ergeben.

Da das Bodenelement des erfindungsgemäßen Zellträgers in erster Linie eine mechanisch stabilisierende Funktion besitzt, sind keine Einschränkungen in Bezug auf das Material des Bodenelements gegeben. Vorteile für eine störungsfreie Bewegung des Zellträgers mit minimalen magnetischen Kräften können sich jedoch ergeben, wenn das Bodenelement aus einem Material mit einer verminderten Haftfähigkeit gegenüber fremden Festmaterialien besteht oder zumindest eine Beschichtung aus einem derartigen Material besitzt. Vorzugsweise wird Polytetrafluorethylen (PTFE) oder ein anderes Kunststoffmaterial mit einer vergleichsweise geringen Haftfähigkeit verwendet. PTFE besitzt zusätzliche Vorteile, da es für die in der Praxis interessierenden Anwendungen bei der Manipulation biologischer Zellen oder Zellkulturen inert ist.

Gemäß einer bevorzugten Ausführungsform des erfindungsgemäßen Zellträgers ist dieser mit dem o. g. Aufnahmeelement ausgestattet, das eine Oberfläche zur Aufnahme der biologischen Probe besitzt, wobei diese Oberfläche in eine andere Richtung weist als das Bodenelement. In der Regel ist das Aufnahmeelement auf einer zum Bodenelement entgegengesetzten Seite des Zellträgers gebildet. Die Bereitstellung des Aufnahmeelements besitzt den Vorteil, dass für die biologische Probe ein definierter und abgegrenzter geometrischer Bereich geschaffen wird, der in Abhängigkeit von den Eigenschaften der Probe und der konkreten Anwendung des Zellträgers in spezifischer Weise gestaltet werden kann. Der Zellträger kann mit der Probe bewegt werden, während diese relativ zum Zellträger im wesentlichen (abgesehen von natürlichen Orientierungsbewegungen, z. B. von biologischen Zellen) unbeweglich ist. Ein weiterer Vorteil des Aufnahmeelements besteht in der Bereitstellung eines Schutzes des Magnetelements gegenüber äußeren Einflüssen. Das Aufnahmeelement bildet eine Abdeckung, die das Magnetelement von einer Umgebungsflüssigkeit oder einer auf dem Zellträger angeordneten Probe trennt.

Das auf der oberen Seite des Zellträgers vorgesehene Aufnahmeelement kann vorteilhafterweise mit einer vorbestimmten Oberflächenform gebildet sein, um die Funktionalität des Zellträgers zu erweitern oder die Oberfläche des Aufnahmeelements für bestimmte Proben anzupassen. Wenn ein ebenes, unstrukturiertes Aufnahmeelement vorgesehen ist, können sich Vorteile für eine universelle Anwendbarkeit des Zellträgers ergeben. Des weiteren wird eine definierte Bezugsfläche geschaffen, die für optische Messungen an der Probe auf dem Zellträger von Vorteil sein kann. Wenn das Aufnahmeelement eine ebene, strukturierte Form besitzt, können sich Vorteile für die Anhaftung und zeitweilige Fixierung der biologischen Probe, insbesondere einer biologischen Zelle ergeben. Es können insbesondere Nano- oder Mikrostrukturen mit typischen Strukturgrößen entsprechend im nm- bis µm-Bereich gebildet sein, welche die Bindung der Zelle an dem Aufnahmeelement beeinflussen. Wenn das Aufnahmeelement eine nicht-ebene, abgestufte Form besitzt, kann vorteilhafterweise eine Vertiefung gebildet werden, die der Positionsbestimmung der Probe und einem lateralen Schutz dient. Der Schutz der Probe auf dem Zellträger kann noch verbessert werden, wenn das Aufnahmeelement einen Hohlraum zur Aufnahme der Probe aufweist.

Das Aufnahmeelement des erfindungsgemäßen Zellträgers kann gemäß einer weiteren Variante der Erfindung in Bezug auf die Wahl eines bestimmten Materials oder die Aufbringung einer Beschichtung modifiziert sein. So kann die Oberfläche des Aufnahmeelements zumindest in einem Teilbereich chemisch so modifiziert sein, dass eine Adhäsion von biologischen Proben, insbesondere von Zellen oder Zellbestandteilen, gefördert oder behindert wird. Zur Adhäsionsförderung kann beispielsweise eine Fibronectin-Beschichtung oder eine Beschichtung zur Bildung von Rezeptor-Liganden-Kombinationen vorgesehen sein. Wenn auf der Oberfläche des Aufnahmeelements spezifische Antikörper vorgesehen sind, an denen bestimmte Zellen spezifisch haften, andere Zellen hingegen nicht haften, können sich Vorteile für eine zellspezifische Bindungsfähigkeit des Aufnahmeelements ergeben.

Wenn das Aufnahmeelement des erfindungsgemäßen Zellträgers einen umlaufenden Rand besitzt, kann vorteilhafterweise eine Abgrenzung der Probe in der Mitte des Aufnahmeelements erreicht werden. Der Rand kann beispielsweise eine adhäsionsmindernde Beschichtung tragen, auf der eine Zelladhäsion behindert oder ausgeschlossen ist. Es kann beispielsweise ein PTFE- oder Poly-HEMA-beschichteter Rand vorgesehen sein. Für praktische Anwendungen ist eine Mindestbreite des beschichteten Randes von 20 µm von Vorteil.

Alternativ kann auf eine adhäsionsmindernde Beschichtung des Randes verzichtet werden, was insbesondere bei Anwendungen von Vorteil ist, wenn sich Zellen auf Zellträgern zu einem Verbund gruppieren sollen und hierzu über Zellträgeroberflächen wachsen oder sich bewegen sollen. In diesem Fall kann eine Mindestbreite des Randes vorzugsweise so gewählt werden, dass Zellen innerhalb der gewünschten Verfahrensdauer (zum Beispiel 0.5 bis 2 h) aufgrund einer eigenen Zellbewegung durch die natürliche Umordnung von Adhäsionskontakten sich nicht vom Zellträger auf die Basisfläche bewegen. Mit einer typischen Eigengeschwindigkeit der Zellen von rd. 100 µm/h wird eine Lateralausdehnung der Oberfläche von mindestens 200 µm bevorzugt.

Wenn gemäß der bevorzugten Ausführungsform der Erfindung das Bodenelement und das Aufnahmeelement übereinander angeordnet sind und der Zellträger einen schichtförmigen Aufbau bildet, ergeben sich Vorteile für die Lagestabilität des Zellträgers. Der schichtförmigen Aufbau ist vorzugsweise so dimensioniert, dass seine Dicke kleiner als 200 µm ist und seine Querschnittsfläche eine laterale Dimension kleiner als 2 mm, besonders bevorzugt kleiner als 1 mm aufweist.

Wenn der Zellträger gemäß einer weiteren Variante der Erfindung einen transparenten Teilbereich aufweist, in dem der Zellträger durchsichtig ist, können sich Vorteile für eine Beobachtung des Zellträgers und z. B. für die Feststellung ergeben, ob sich eine Probe auf dem Zellträger befindet.

Ein weiterer Vorteil des erfindungsgemäßen Zellträgers besteht darin, dass die Aufnahme-, Magnet- und Bodenelemente mit großer Flexibilität an die jeweilige Anwendung angepasst gebildet sein können. Es kann insbesondere vorgesehen sein, dass das Magnetelement und das Bodenelement ein gemeinsames Bauteil bilden. In diesem Fall wird die stabile Auflage des Zellträgers durch die untere Seite des Magnetelements bereitgestellt. Entsprechend kann das Magnetelement die Funktion des Aufnahmeelements übernehmen. Allgemein kann ein erfindungsgemäßer Zellträger ausschließlich aus dem Magnetelement bestehen, dessen untere Oberfläche die Auflage auf der Basisfläche und dessen obere Oberfläche die Aufnahme für die Probe bilden, wobei in diesem Fall ein besonders einfacher Aufbau des Zellträgers gegeben ist.

Alternativ können das Magnetelement einerseits und die Aufnahme- und/oder Bodenelemente andererseits getrennte Bauteile bilden. Beispielsweise kann das Magnetelement nach Art eines herkömmlichen Magnetbeads mit der Probe verbunden sein, die auf dem Aufnahmeelement angeordnet ist.

Das mindestens eine Magnetelement des erfindungsgemäßen Zellträgers ist zur Erzeugung eines Magnetfeldes oder zur Wechselwirkung mit einem extern erzeugten Magnetfeld eingerichtet und umfasst hierzu allgemein ein magnetisches oder magnetisierbares Material. Erfindungsgemäß kann das Magnetelement ein paramagnetisches, ferromagnetisches und/oder diamagnetisches Material aufweisen. Besondere Vorteile für eine wirksame Manipulation des Zellträgers mit einer externen magnetischen Stelleinrichtung ergeben sich bei einer hohen Eigenmagnetisierung oder Magnetisierbarkeit, so dass das Magnetelement vorzugsweise Metalle wie z.B. Fe oder Ni, oder Legierungen, wie z.B. FeSi (96 % Fe, 4 % Si), Permalloy oder Mu-Metall (Ni/Fe/Cu/Cr) aufweisen. Des weiteren kann die Verwendung von paramagnetischen Materialien wie z.B. Pt oder von diamagnetischen Materialien wie z.B. Bi vorgesehen sein.

Weitere Vorteile für eine starke magnetische Wechselwirkung und die Flexibilität bei der Materialauswahl für das Magnetelement können sich ergeben, wenn als Magnetelement eine Induktionseinrichtung vorgesehen ist. Vorteilhafterweise können mit Mikrostrukturierungstechniken Induktivitäten z.B. in Schichtform auf festen Oberflächen wie der Unterseite des Aufnahmeelements gebildet werden, die eine geringe Bauhöhe und dennoch eine hohe magnetische Wechselwirkung zeigen. Als Magnetelement kann beispielsweise eine Spule mit mindestens einer Windung und gegebenenfalls mit einem Spulenkern, z.B. aus Fe, vorgesehen sein. Vorteilhafterweise bestehen keine Beschränkungen in Bezug auf die Spulenform. Wenn eine Spule ringförmig als Zylinderspule gebildet ist, können sich Vorteile für eine hohe Magnetfeldstärke ergeben. Eine in einer Bezugsebene parallel zu den Aufnahme- und Bodenelementen gewickelte Spule besitzt den Vorteil, dass die Bauhöhe des Zellträgers gering gehalten werden kann.

Ein weiterer Vorteil der Erfindung besteht darin, dass in Bezug auf die Form des Magnetelements und seiner Anordnung im Zellträger keine Beschränkungen bestehen. Vielmehr kann die Gestaltung des Magnetelements in Abhängigkeit von der konkreten Anwendung des Zellträgers gewählt werden. Wenn das Magnetelement beispielsweise eine parallel zum Bodenelement verlaufende, durchgehende Schicht aus dem magnetisierbaren oder magnetischen Material ist, kann vorteilhafterweise eine hohe magnetische Kraft erreicht werden.

Alternativ kann das Magnetelement mehrere Teilelemente umfassen, die im Körper des Zellträgers verteilt sind, wobei sich Zusatzfunktionen des Magnetelements realisieren lassen. Wenn die Teilelemente (oder ein einzelnes Magnetelement) mit einem Abstand von mindestens einem seitlichen Rand des Zellträgers angeordnet sind, können gegenseitige Wechselwirkungen zwischen benachbarten Zellträgern vermindert werden. Hierzu ist das Magnetelement vorzugsweise in der Mitte des Zellträgers lokalisiert. Alternativ können das Magnetelement oder mehrere Teilelemente gerade am Rand des Zellträgers angeordnet sein, um eine Wechselwirkung nicht nur mit einer externen magnetischen Stelleinrichtung, sondern auch zwischen benachbarten Zellträgern zu erzielen. Beispielsweise kann der Zellträger für eine Bewegung in eine bestimmte Bewegungsrichtung ausgelegt sein. Wenn Teilelemente des Magnetelements am vorderen und/oder hinteren Rand in Bezug auf die Bewegungsrichtung angeordnet sind, kann je nach Ausrichtung von Magnetpolen eine kettenartige Verknüpfung benachbarter Zellträger oder gerade die Schaffung eines Sicherheitsabstandes zwischen benachbarten Zellträgern erreicht werden. Durch die Teilelemente kann eine magnetische Orientierung des Zellträgers definiert werden. Wenn die magnetische Orientierung mit einer geometrischen Vorzugsrichtung des Zellträgers zusammenfällt, kann dessen Manipulation unter optischer Kontrolle erleichtert werden.

Gemäß einer weiteren, besonders vorteilhaften Ausführungsform der Erfindung können das Magnetelement oder die Teilelemente des Magnetelements mindestens eine Datenspeicherzelle bilden. Vorteilhafterweise ermöglicht dies das Einschreiben von Identifizierungsinformationen oder weiteren Daten in den Zellträger, die den Zellträger oder die auf diesem befindliche Probe charakterisieren.

Ein weiterer, wichtiger Vorteil der Erfindung besteht darin, dass auch in Bezug auf die laterale Außenform des Zellträgers keine Beschränkungen bestehen. Es kann zum Beispiel eine Au-ßenform gebildet werden, die in verschiedene Richtungen zueinander komplementär geformte Teilstücke aufweist. Vorteilhafterweise können dadurch mehrere Zellträger eng zusammengesetzt werden, um einen erfindungsgemäßen Kulturträger (siehe unten) zu bilden und/oder bestimmte Reaktionen zwischen den Proben zu erzielen. Die Außenform des Zellträgers ist allgemein die Form des seitlichen Randes des Zellträgers in einer Bauhöhe, in welcher der Zellträger seine maximale Ausdehnung besitzt. Bei konus- oder pyramidenförmigen Zellträgern wird die Außenform beispielsweise durch den Umriss des Bodenelements gebildet. Alternativ kann, wenn das Aufnahmeelement und/oder das Magnetelement über dem Bodenelement einen Vorsprung bildet, die Außenform durch den Umriss des Aufnahmeelements und/oder das Magnetelement gebildet werden.

Wenn die Außenform durch ein Polygon darstellbar ist und entsprechend durch mindestens drei gerade Abschnitte begrenzt wird, können sich Vorteile für ein abstandfreies Zusammensetzen der Zellträger zu einem Kulturträger ergeben. Es wird vorzugsweise eine Außenform gewählt, die eine lückenlose Flächenpackung von gleich geformten Zellträgern ermöglicht, was beispielsweise bei einer regelmäßig hexagonalen oder dreieckigen Außenform der Fall ist. Lückenlose Anordnungen einer Vielzahl von Zellträgern ermöglichen vorteilhafterweise den Aufbau geschlossener Substratoberflächen für die Kultivierung von Zellen.

Alternativ kann bei bestimmten Anwendungen bevorzugt sein, dass sich die Zellträger gerade nicht lückenlos zusammensetzen lassen oder dass eine besonders große Fläche des Aufnahmeelements gebildet werden soll. In diesen Fällen wird eine abgerundete Außenform des Zellträgers realisiert. Es kann beispielsweise eine kreisrunde oder eine ellipsenförmige Au-ßenform vorgesehen sein.

Gemäß einer weiteren Modifizierung der Erfindung kann ein Zellträger mit mindestens einem seitlichen Stützelement ausgestattet sein, das einen Schutz gegen ein unerwünschtes Verkippen des Zellträgers bietet. Ein derartiger Schutz kann beispielsweise von Vorteil sein, wenn äußere mechanische Kräfte, wie z.B. Strömungskräfte in einem Kulturmedium oder magnetische Störkräfte auftreten. Ein Stützelement ist vorzugsweise ein seitlich vom Körper des Zellträgers abstehender Stab oder Streifen, der starr oder biegsam ausgebildet sein kann. Gemäß einer vorteilhaften Variante der Erfindung können Stützelemente eine Zusatzfunktion beim Zusammensetzen von Zellträgern zu einem erfindungsgemäßen Kulturträger erfüllen, indem sich Stützelemente von benachbarten Zellträgern miteinander verhaken. Am Körper des Zellträgers können des weiteren Ausnehmungen vorgesehen sein, die zu den Stützelementen komplementär geformt sind, um ein Ineinandergreifen benachbarter Zellträger zu ermöglichen.

Wenn der erfindungsgemäße Zellträger gemäß einer weiteren Ausführungsform der Erfindung mindestens ein Identifizierungselement trägt, können sich Vorteile für die Überwachung einer Vielzahl von Zellträgern z.B. in einer erfindungsgemäßen Manipulationseinrichtung (siehe unten) ergeben. Als Identifizierungselement kann beispielsweise ein optischer Datenträger nach Art eines Strich-Codes oder der oben genannte magnetische Datenspeicher vorgesehen sein.

Allgemein ist die Anwendung der Erfindung nicht auf bestimmte Größenverhältnisse beschränkt. Besondere Vorteile für die beabsichtigten biologischen und medizinischen Anwendungen ergeben sich jedoch, wenn der Zellträger als miniaturisiertes Bauteil hergestellt wird, das zur Aufnahme einzelner Zellen oder einzelner Zellgruppen eingerichtet ist. Die zur Probenaufnahme vorgesehene Oberfläche des Zellträgers (insbesondere: des Aufnahmeelements des Zellträgers) besitzt vorzugsweise eine freie Fläche, die zur adhärenten Anhaftung maximal einer Zelle eingerichtet ist. Hierzu werden typischerweise laterale Dimensionen (zum Beispiel Durchmesser oder Kantenlänge) der Oberfläche im Bereich von 10 µm bis 1 cm, insbesondere von 10 µm bis 5000 µm gewählt. Die auf der Unterseite des Zellträgers gebildete Auflage besitzt vorzugsweise ebenfalls eine Dimension in diesem Bereich. Für eine stabile Positionierung und/oder Bewegung des Zellträgers auf der Basisfläche ist die Höhe des Zellträgers vorzugsweise kleiner als die kleinste laterale Dimension der zur Probenaufnahme vorgesehenen Oberfläche gewählt. Die Höhe entspricht dem senkrechten Abstand zwischen der Oberfläche des Aufnahmeelements und der durch das Bodenelement gebildeten Auflage. Sie wird vorzugsweise im Bereich von 0.5 µm bis 2000 µm, insbesondere von 1 µm bis 1000 µm gewählt. Durch die Bereitstellung der miniaturisierten Zellträger wird ein Werkzeug zur zellspezifischen Probenmanipulation geschaffen, das überraschenderweise mit hoher Zuverlässigkeit und Selektivität mit makroskopischen Stelleinrichtungen bedienbar ist.

Vorrichtungsbezogen basiert die Erfindung gemäß einem zweiten Aspekt auf der allgemeinen technischen Lehre, einen Kulturträger zur Aufnahme biologischer Zellen bereitzustellen, der eine Vielzahl der erfindungsgemäßen Zellträger umfasst. Wenn die Zellträger gruppenweise zusammengesetzt werden, wird ein Substrat für eine Vielzahl biologischer Zellen geschaffen, das zur Kultivierung der Zellen verwendet werden kann und hier entsprechend als Kulturträger bezeichnet wird. Ein wesentlicher Vorteil des erfindungsgemäßen Zellträgers besteht in der Schaffung eines extrem flexiblen Substrats, das in Abhängigkeit von der konkreten Anwendung in den verschiedensten Formen zusammengestellt, aufgetrennt, in Teilen kombiniert und anderweitig prozessiert werden kann.

Gemäß einer ersten Variante sind die Zellträger des Kulturträgers nebeneinander in seitlichem Kontakt angeordnet. Die Zellträger sind in diesem Fall nicht miteinander verbunden. Es ist lediglich eine gegenseitige, seitliche Berührung vorgesehen. Überraschenderweise reicht dieser lose Zusammenschluss von Zellträgern aus, dass Zellen auf benachbarten Zellträgern in mechanischen oder stofflichen Kontakt treten und/oder eine Kultivierung der Zellen auftritt. Alternativ können die Zellträger des Kulturträgers miteinander verankert sein. Ein Verbund von Zellträgern besitzt den Vorteil einer erhöhten Stabilität. Die Zellträger können auch im Verbund voneinander lösbar angeordnet oder alternativ unlösbar verbunden sein.

Gemäß einer bevorzugten Ausführungsform der Erfindung bilden die Aufnahmeelemente der zusammengesetzten Zellträger eine ebene Kulturträgeroberfläche. Diese besitzt den Vorteil einer Kompatibilität mit herkömmlichen Kultivierungssubstraten. Alternativ können die Zellträger eine nicht-ebene, z.B. gekrümmte oder abgestufte Kulturträgeroberfläche bilden. In diesem Fall können sich Vorteile für die Schaffung von Zellkulturen mit bestimmten Geometrien ergeben, wie es im so genannten "Tissue Engineering" erwünscht sein kann.

Vorrichtungsbezogen basiert die Erfindung gemäß einem dritten Aspekt auf der allgemeinen technischen Lehre, eine Manipulationseinrichtung für biologische Proben bereitzustellen, die eine Basisfläche zur Positionierung mindestens eines erfindungsgemäßen Zellträgers und eine Stelleinrichtung zur Ausübung einer magnetischen Kraft auf den mindestens einen Zellträger aufweist. Mit der Manipulationseinrichtung werden Nachteile insbesondere von herkömmlichen fluidischen Mikrosystemen mit berührungslosen Partikelmanipulationen in elektrischen Feldern dahingehend überwunden, dass Zellmanipulationen an Zellen durchführbar sind, die einen adhärenten Oberflächenkontakt besitzen und dennoch einzeln beweglich sind. Die Zellmanipulationen sind mit einer hohen Stabilität und Reproduzierbarkeit realisierbar. Für einen sicheren Betrieb ist es nicht erforderlich, Trägerströmungen oder elektrische Felder aufrechtzuerhalten. Zellträger mit Proben und/oder biologisch wirksamen Substanzen können mit vorgebbaren Raum- und Zeitprogrammen zum Beispiel positioniert, bewegt und zur Wechselwirkung gebracht werden. Es können Zeitschemata realisiert werden, die an die praktisch interessierenden Kultivierungs- und Wechselwirkungszeiten im Minuten- bis in den Stundenbereich angepasst sind.

Die Basisfläche ist Teil einer Kultivierungseinrichtung, wie zum Beispiel eines Kulturgefäßes oder eines fluidischen Mikrosystems. Die Bewegung der Zellträger kann über kleinste Magnetquellen realisiert werden, die als Kraftelemente von der äußeren Umgebung der Manipulationseinrichtung her über die Basisfläche wirksam sind, wodurch eine vollständige Sterilität der Zellträger garantiert werden kann. Die Kraftelemente können so geformt werden, dass eine Beeinflussung des ggf. vorhandenen Nachbar-Zellträgern minimiert wird. Durch Bewegung der außen befindlichen Kraftelemente werden die Zellträger im Inneren eines Kulturgefäßes oder Mikrosystem mitgenommen. Hierzu können z. B. programmierbare x-y-z Manipulatoren verwendet werden, die durch Wegfahren von der Kanalfläche die Kräfte auf den Zellträger unterbrechen können. Ein paralleler Betrieb oder eine sehr rasche Ansteuerung derartiger Manipulatoren sorgt für die geordnete Bewegung mehrerer Zellträger gleichzeitig.

Die Basisfläche ist allgemein Teil eines Bauteils zur Halterung der Zellträger, das vorzugsweise schicht- oder plattenförmig gebildet ist und als Halteplatte (insbesondere Boden- oder Deckplatte) bezeichnet wird. Wenn die Stelleinrichtung auf einer Rückseite der Halteplatte angeordnet ist, die zu der Basisfläche mit den Zellträgern entgegengesetzt ist, kann die Basisfläche vorteilhafterweise eine freie Plattform bilden. Auf der Plattform können Verfahren insbesondere zur Behandlung oder Bearbeitung von einzelnen Zellen oder Zellkulturen gut beobachtet und ggf. durch zusätzliche Maßnahmen beeinflusst werden.

Gemäß einer bevorzugten Ausführungsform der Erfindung enthält die Basisfläche zumindest in Teilbereichen ein magnetisches oder magnetisierbares Material, dessen Wechselwirkung mit dem Magnetelement des mindestens einen Zellträgers schwächer als die Wechselwirkung der Stelleinrichtung mit dem Magnetelement ist. Damit kann ein passives, stabiles Fixieren des Zellträgers auf der Basisfläche erreicht werden, selbst wenn die Stelleinrichtung entfernt oder abgeschaltet ist.

Wenn gemäß einer weiteren Variante die Basisfläche eine ebene Oberfläche besitzt, können sich Vorteile für eine allseitige Verschiebbarkeit von Zellträgern ergeben. Alternativ kann die Basisfläche eine gewölbte Form besitzen. Dies ermöglicht vorteilhafterweise eine Prozessführung im Inneren von geschlossenen Behältnissen, wie zum Beispiel in Gefäßen oder Hohlzylindern.

Die Funktionalität der Manipulationseinrichtung kann erheblich erweitert werden, wenn die Basisfläche eine Oberflächenstruktur mit Vorsprüngen und/oder Vertiefungen besitzt. In diesem Fall wird eine Topographie geschaffen, mit der bestimmte Teilbereiche der Basisfläche für bestimmte Prozessaufgaben optimierbar sind. Die Oberflächenstruktur umfasst als Formelemente vorzugsweise mindestens eine Freifläche, einen Kanal, eine Verzweigung, eine Weiche, eine Ringbahn und/oder Höfe. Eine Freifläche ist ein unstrukturierter Teilbereich, der sich besonders für Zellkultivierungen mit einer Vielzahl von Zellträgern eignet. Ein Kanal ist ein gerader oder gekrümmter, lang gestreckter Teilbereich der Basisfläche mit einer Längsrichtung, welcher der Abgrenzung der Bewegungsbahn von Zellträgern zwischen seitlichen Kanalwänden dient. Spezielle Kanalformen zeichnen sich durch eine Aufteilung in oder Verbindung von mindestens zwei Kanälen (Verzweigung, Weiche) oder eine umlaufend geschlossene Kanalform (Ringbahn) aus. Kurze Kanäle mit einem geschlossenen Ende bilden so genannte Höfe und dienen als Parkpositionen für einzelne Zellträger oder Zellträgergruppen.

Gemäß einer weiteren Ausführungsform der Erfindung kann die Basisfläche mindestens einen transparenten Bereich aufweisen. Vorteilhafterweise wird dadurch eine Beobachtung des Zellträgers und des Magnetelements und/oder der Relativpositionen beider Teile insbesondere während des Betriebs der Manipulationseinrichtung ermöglicht.

Eine erfindungsgemäße Manipulationseinrichtung kann mit zwei Halteplatten ausgestattet sein, die zwei Basisflächen bilden, deren freie Oberflächen zueinander weisend mit einem Abstand angeordnet sind und die beide der Aufnahme von Zellträgern dienen. Zwischen den Basisflächen wird vorteilhafterweise ein Reaktionsraum geschaffen, an dessen beiden Wänden die erfindungsgemäßen Zellträger manipulierbar sind. Der Abstand zwischen den vorzugsweise geometrisch gleich geformten Basisflächen kann so gewählt werden, dass Proben auf Zellträger, die sich auf den Basisflächen gegenüber stehen, miteinander in Wechselwirkung treten können.

Wenn die Basisfläche der erfindungsgemäßen Manipulationseinrichtung mit einer Flüssigkeit wie zum Beispiel einem Kulturmedium überdeckt ist, können sich Vorteile für die Einstellung von Kultivierungsbedingungen ergeben.

Die Stelleinrichtung der Manipulationseinrichtung weist mindestens ein Kraftelement, mit dem eine magnetische Wechselwirkung mit dem Zellträger erzeugt werden kann. Das Kraftelement enthält in Abhängigkeit vom Magnetelement des Zellträgers einen Permanentmagneten, einen Paramagneten, einen Ferromagneten, einen Diamagneten, einen elektrisch anregbaren Magneten (Induktivität) und/oder eine magnetisch Flüssigkeit. Vorteilhafterweise kann jeder dieser Kraftelementtypen miniaturisiert hergestellt werden. Dies ermöglicht die Anpassung der Kraftelemente an die Größen der Zellträger und die Bereitstellung einer Vielzahl von Kraftelementen auf engem Raum.

Grundsätzlich könnten die Kraftelemente ortsfest angeordnet sein, wobei eine Verschiebung von Zellträgern durch aufeinanderfolgende Ansteuerung benachbarter Kraftelemente erreicht werden kann. Bevorzugt ist jedoch eine Ausführungsform der Erfindung, bei der die Manipulationseinrichtung mit einer Verschiebeeinrichtung ausgestattet ist, mit der die Kraftelemente einzeln relativ zur Basisfläche verschiebbar sind.

Wenn gemäß einer weiteren Ausführungsform der Erfindung die Manipulationseinrichtung mit einer Monitoreinrichtung ausgestattet ist, können sich Vorteile für die Beobachtung der Zellträger und die Steuerung von Verfahren mit den Zellträgern ergeben. Als Monitoreinrichtung ist beispielsweise ein Mikroskop vorgesehen.

Verfahrensbezogen basiert die Erfindung gemäß einem weiteren Aspekt der Erfindung auf der allgemeinen technischen Lehre, zur Manipulation einer biologischen Probe diese auf einem Zellträger anzuordnen, der lagestabil auf einer Basisfläche positioniert ist, und den Zellträger mit einer magnetischen Kraft zu bewegen oder an einem vorbestimmten Ort zu positionieren. Der Vorteil dieses Verfahrens besteht in der Bewegung adhärent gebundener Zellen ohne eine Störung ihres Bindungszustandes. Als Zellträger wird vorzugsweise der oben beschriebene, erfindungsgemäße Zellträger verwendet. Die Manipulation erfolgt vorzugsweise mit der oben beschriebenen, erfindungsgemäßen Manipulationseinrichtung.

Gemäß einer bevorzugten Ausführungsform der Erfindung werden Proben auf verschiedenen Zellträgern auf der Basisfläche zu einer gegenseitigen Wechselwirkung miteinander oder mit Wirkstoffen gebracht. Zellträger werden zueinander benachbart angeordnet, so dass Zellen und/oder Zellbestandteile auf den Zellträgern miteinander oder mit Wirkstoffen in zum Beispiel mechanische oder stoffliche Wechselwirkung treten. Wenn sich Zellen und/oder Zellbestandteile auf benachbarten Zellträgern gegenseitig berühren, können vorteilhafterweise Membrankontakte erzeugt werden.

Die Erfindung besitzt die folgenden weiteren Vorteile. Es wird erstmalig eine praktikable Möglichkeit geschaffen, Zellen in einer Nährlösung in adhärentem Zustand zum Beispiel in Mikrokanälen unter sterilen Bedingungen auf individuellen Zellträgern zu bewegen, ohne dass dabei die erforderlichen Kräfte an den Zellen selbst angreifen. Darüber hinaus wird eine Gruppierung der Zellträger, ihre erneute Trennung oder feste Kopplung, eine Bewegung von Zellen von Zellträger zu Zellträger, ein Überwachsen von Zellträgern mit Zellen, eine Musterbildung und wiederholte Gruppierung von Zellträgern und auch eine Einbringung und Entnahme der Zellträger und Zellträgergruppen mit Zellen aus einer Manipulationseinrichtung wie zum Beispiel einem Mikrokanalsystemen ermöglicht. Ein wesentlicher Vorteil der Erfindung ist die definierte und vorher festlegbare Orientierung und Lokalisation der Zelle in der Manipulationseinrichtung und relativ zueinander. Zufällige Faktoren können weitestgehend ausgeschlossen werden. Durch die Gestaltungsfreiheit bei der Schaffung eines erfindungsgemäßen Kulturträgers kann die Geometrie und das Material einer Besiedlungsfläche für die Zellanhaftung weitgehend frei gewählt werden.

Des Weiteren wird eine Beobachtbarkeit und auch eine Abbildung der adhärenten, zum Beispiel adhärent wachsenden Zellen ermöglicht. Es können alle bekannten Mikroskopieverfahren angewendet werden, die Abbildung kann mit höchster mikroskopischer Auflösung erfolgen.

Die Manipulation der Zellträger ermöglicht erstmalig eine bislang nicht realisierte, freie Bewegung entgegen einer Strömung oder sogar in unterschiedliche Richtungen zum Beispiel in einem durchströmten Kanal einer Manipulationseinrichtung. Zellen können auch gegen eine Oberflächenspannung einer Ausgangsöffnung aus der Manipulationseinrichtung bewegt werden.

Ein weiterer Vorteil besteht darin, dass eine parallele Manipulation von mehreren Zellträgern und eine Bewegung der Zellträger an möglichst vielen Wandflächen einer Manipulationseinrichtung möglich ist, so dass auch verschiedenen Zellträgern mit Zellen an einander vorbei oder sogar gegenläufig bewegt werden können. Dabei kann die Präzision der Bewegung im Mikro- und Nanometerbereich liegen und sehr schnelle (mm/s) bis zu sehr langsamen (µm/h) Bewegungen erlauben.

Vorteilhafte Ausführungsformen und Anwendungen der Erfindung werden im folgenden unter Bezug auf die beigefügten Zeichnungen beschrieben. Es zeigen:
- Figur 1:: Schnittansichten verschiedener Ausführungsformen von erfindungsgemäßen Zellträgern,
- Figuren 2 und 3:: Perspektivansichten weiterer Ausführungsformen von erfindungsgemäßen Zellträgern,
- Figur 4:: Draufsichten verschiedener Ausführungsformen von erfindungsgemäßen Kulturträgern,
- Figur 5:: eine Schnittansicht einer weiteren Ausführungsform eines erfindungsgemäßen Kulturträgers,

- Figur 6:: Illustrationen einer Ausführungsform einer erfindungsgemäßen Manipulationseinrichtung,
- Figuren 7 und 8:: Seitenansichten weiterer Ausführungsformen von erfindungsgemäßen Manipulationseinrichtungen,
- Figuren 9 und 10:: Illustrationen von Ausführungsformen der erfindungsgemäßen Zellmanipulierungs-Verfahren,
- Figuren 11 und 12:: Illustrationen der Kraftausübung an erfindungsgemäßen Zellträgern,
- Figuren 13 und 14:: Draufsichten weiterer Ausführungsformen von erfindungsgemäßen Manipulationseinrichtungen, und
- Figuren 15 und 16:: Illustrationen von Ausführungsformen erfindungsgemäßer Manipulationseinrichtungen mit gewölbten Basisflächen.

Die Erfindung wird im folgenden unter beispielhaftem Bezug auf schematisch illustrierte Grundformen von Zell- und Kulturträgern beschrieben. Es wird betont, dass die Umsetzung der Erfindung nicht auf die illustrierten Beispiele beschränkt ist, sondern entsprechend insbesondere mit abgewandelten Formen, Größen und Materialien, die an die konkrete Aufgabe einer Anwendung angepasst sind, realisiert werden kann.

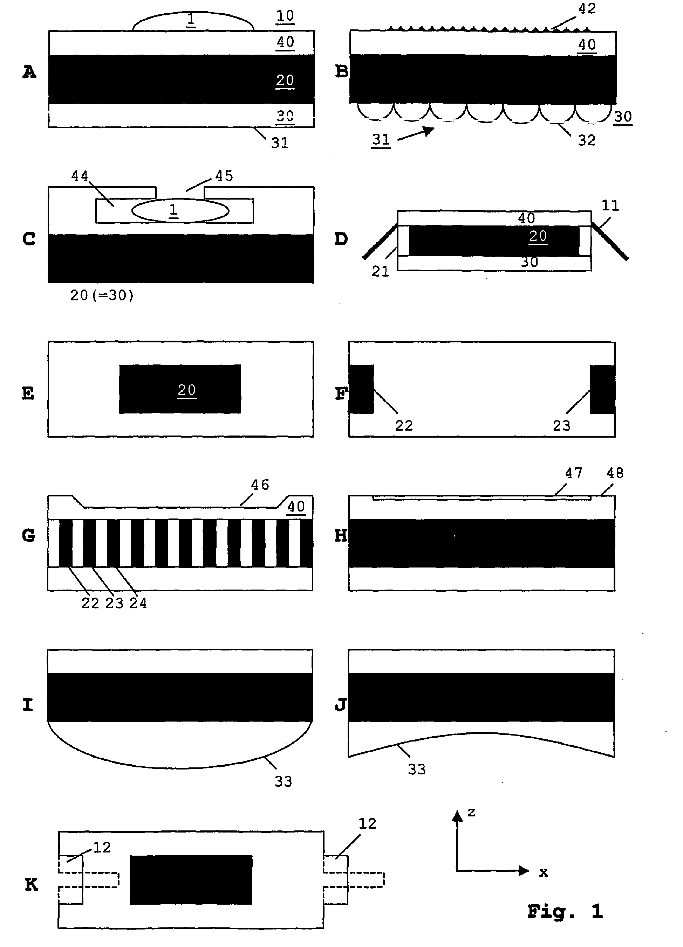

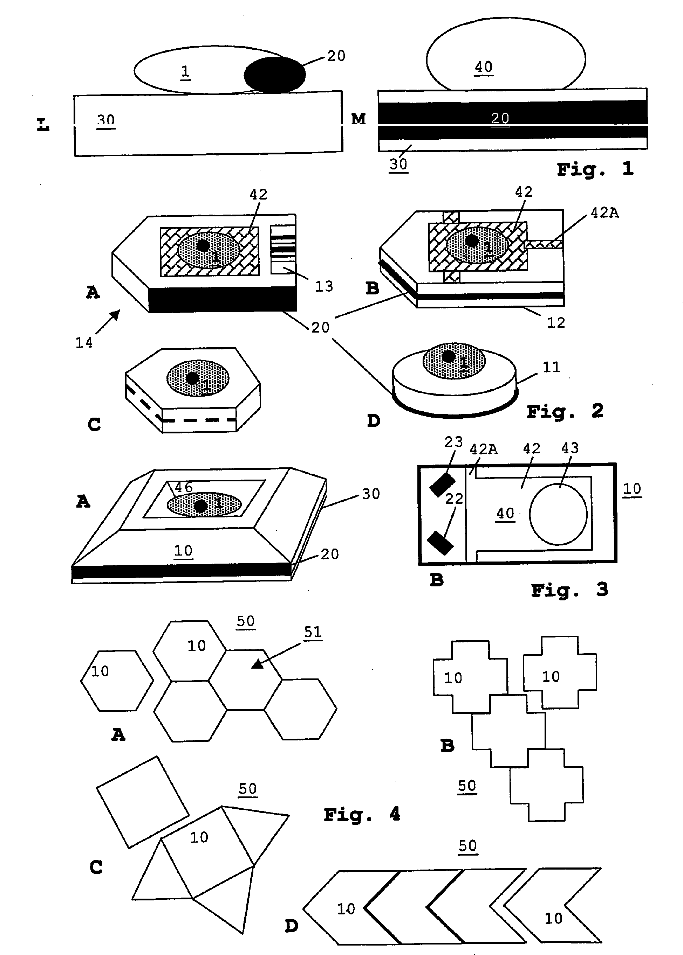

Figur 1 zeigt in den Teilbildern A bis M Vertikal-Schnittansichten von erfindungsgemäßen Zellträgern 10, die jeweils das Magnetelement 20, das Bodenelement 30 und das Aufnahmeelement 40 umfassen. Diese Elemente können verschiedene Teile umfassen oder durch gemeinsame Teile realisiert sein. Der Zellträger 10 besitzt erfindungsgemäß allgemein die Gestalt einer Platte oder Scheibe, deren Dicke (oder: Höhe) geringer als die minimale laterale Ausdehnung ist. Die Plattenform erstreckt sich im wesentlichen in einer x-y-Ebene, die parallel zur Ausrichtung einer ebenen Basisfläche einer Manipulationseinrichtung (siehe unten) verläuft. Die Elemente 20, 30 und 40 sind bei den meisten Beispielen im wesentlichen als Stapel übereinander angeordnet. Die Herstellung der Zellträger erfolgt mit an sich bekannten mikromechanischen oder mikrochemischen Methoden der Mikrostrukturtechnik, zum Beispiel aus Silizium, Glas und/oder Kunststoff.

Gemäß Figur 1A besitzen das Magnetelement 20, das Bodenelement 30 und das Aufnahmeelement 40 jeweils eine Schichtform. Das Magnetelement 20 enthält Eisen und besitzt eine Dicke im Bereich von 100 nm bis 500 µm, vorzugsweise im Bereich von 1 µm bis 100 µm, z. B. 40 µm. Das Bodenelement 30 besteht aus einer Schicht, die eine ebene, flächige Auflage 31 bildet, zum Beispiel aus PTFE. Die Auflage 31 bildet vorteilhafterweise eine Schutzschicht zwischen dem Magnetelement 20 und der Umgebung. Die PTFE-Schicht besitzt eine Dicke von einigen µm. Das Aufnahmeelement 40 ist eine Kunststoffschicht, zum Beispiel aus Polyethylen (PE) oder Polyurethan (PU), mit einer Dicke von 0.1 µm bis 100 µm. Auf dem Aufnahmeelement 40 ist eine Zelle 1 angeordnet. Die Elemente 20, 30 und 40 besitzen gemäß Figur 1A in der x-y-Ebene die gleiche Außenform, so dass sie einen geraden Stapel bilden. Die Querschnittsfläche senkrecht zur z-Richtung ist zum Beispiel kreisrund mit einem Durchmesser von 300 µm. Die laterale Dimension des Zellträgers liegt zum Beispiel im Bereich von 10 µm bis 1 cm und ist vorzugsweise kleiner als 2 mm, besonders bevorzugt kleiner als 1 mm, während seine Höhe einen Wert zum Beispiel im Bereich von 1 µm bis 1000 µm besitzt und vorzugsweise weniger als 200 µm beträgt.

Das Bodenelement 30 und das Aufnahmeelement 40 können Oberflächen aufweisen, die beide aus dem selben Material (z. B. PE oder PU) hergestellt sind, so dass der Aufbau des Zellträgers in z-Richtung symmetrisch zu einer Mittelebene wird. Diese Symmetrie kann für die Handhabung der Zellträger von Vorteil sein.

Die Beladung des Aufnahmeelements 40 mit der Zelle 1 oder mit Zellbestandteilen kann mit an sich bekannten Dispensiertechniken, zum Beispiel mit einer Pipette erfolgen. Alternativ kann ein Aufwandern von benachbarten Zellträgern durch eine Eigenbewegung der Zelle vorgesehen sein. Falls auf dem Aufnahmeelement 40 als Zellbestandteil nur eine Zellspur angeordnet wird, wie sie von wandernden Zellen auf Substraten als Materialrückstand hinterlassen wird, kann die Beladung durch ein Überwandern des Aufnahmeelements 40 erfolgen.

Figur 1B illustriert den Zellträger mit einem Bodenelement 30, das eine Vielzahl halbkugel- oder halbzylinderförmigen Strukturen mit Auflagepunkten 32 umfasst, die in einer gemeinsamen Ebene liegen und die Auflage 31 bilden, und eine im Vergleich zu Figur 1A verminderte Reibung auf einer Basisfläche besitzt. Des Weiteren zeigt Figur 1B, dass das Aufnahmeelement 40 in der Mitte als strukturierte Oberfläche 42 eine Aufrauung mit typischen Strukturdimensionen von z. B. 0.01 µm bis 100 µm besitzt.

Gemäß Figur 1C ist das Aufnahmeelement mit einem Hohlraum 44 gebildet, der eine Öffnung 45 aufweist. Durch die Öffnung 45 kann eine Zelle 1 im Hohlraum 44 abgelegt werden. Figur 1C illustriert des Weiteren, dass die Funktion des Bodenelements 30 vom Magnetelement 20 übernommen wird.

Die in Figur 1D illustrierten Stützelemente 11 dienen insbesondere einem Kippschutz des Zellträgers. Die Stützelemente 11 sind als seitlich abstehende, zum Boden weisende Streifen gebildet, die zum Beispiel einstückig mit dem Material des Aufnahmeelements 40 oder des Bodenelements 30 gebildet sind. Das Magnetelement 20 besitzt bei diesem Beispiel eine Schichtform, die nicht durch den gesamten Körper des Zellträgers verläuft, sondern zum Rand hin von der Umgebung durch eine Schutzschicht 21, zum Beispiel aus Kunststoff getrennt ist. Die Schutzschicht 21 kann ebenfalls einstückig mit dem Aufnahmeelement 40 gebildet sein.

Erfindungsgemäß kann vorgesehen sein, dass die Stützelemente 11 teilweise oder komplett die Funktion des Bodenelements 30 übernehmen. So kann das Bodenelement 30 gemäß Figur 1B nur einen oder zwei Auflagepunkte 32 aufweisen, die gemeinsam mit mindestens einem weiteren Stützelement 11 die gewünschte stabile Auflage bilden. Des Weiteren kann der Zellträger komplett auf mindestens drei Stützelementen 11 ruhen.

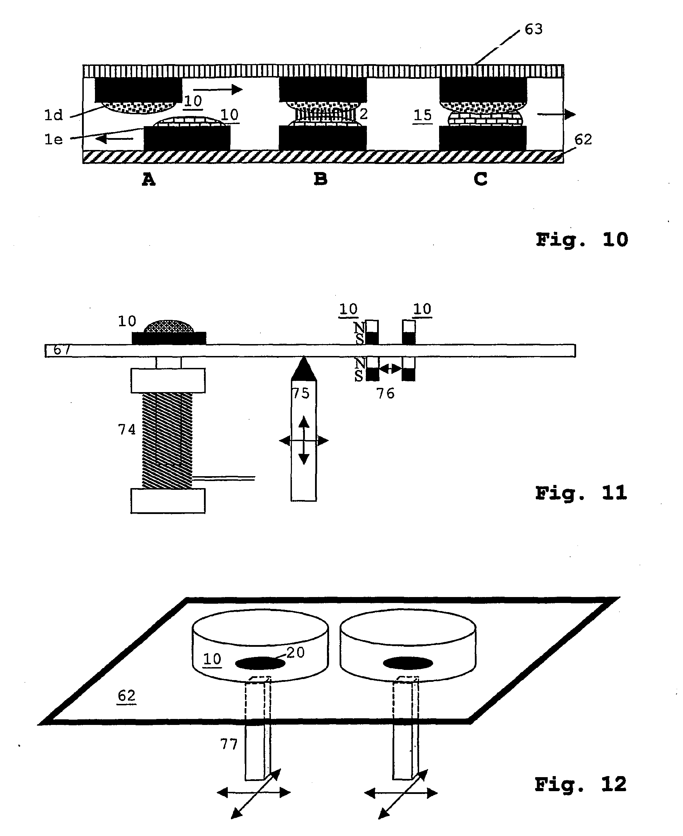

Wenn die Schutzschicht 21 besonders dick gewählt wird, ergibt sich die in Figur 1E gezeigte mittige Anordnung des Magnetelements 20 im Körper des Zellträgers. Der magnetisierbare oder magnetische Bereich ist auf einen Zentralteil des Körpers beschränkt, so dass magnetische Wechselwirkungen zwischen den benachbarten Zellträgern so klein gehalten oder ausgeschlossen werden können (siehe auch Figur 12).

Gemäß den Figuren 1F und 1G umfasst das Magnetelement 20 mehrere, im Zellträger 10 verteilte Teilelemente 22, 23, 24. Die Teilelemente 22, 23 an Rändern des Zellträgers (Figur 1F) können die Wechselwirkung mit benachbarten Zellträgern beeinflussen (Kettenbildung oder Abstoßung je nach Verteilung und Polung der Teilelemente). Figur 1F zeigt auch, dass die Aufnahme- und Bodenelemente einstückig aus einem Material, zum Beispiel in Form einer Kreisscheibe oder eines flachen Quaders, hergestellt sein können, in das die Teilelemente 22, 23 seitlich eingelassen sind. Alternativ können die Teilelemente 22, 23 schichtförmig auf dem Zellträgerkörper aufgebracht sein (siehe Figuren 2 A, C).

Die Teilelemente 22, 23, 24 an Rändern und im Innern des Zellträgers (Figur 1G) können als Datenspeicher verwendet werden. Sie umfassen zum Beispiel getrennt magnetisierbare Partikel oder Domänen eines Magnetelements, deren Magnetisierungsrichtungen einzeln mit einem externen Magnetfeld einstellbar sind.

Figur 1G illustriert eine Strukturierung des Aufnahmeelements 40 mit einer Abstufung, so dass eine Vertiefung 46 gebildet wird. Die Vertiefung 46 ist zum Beispiel mit an sich bekannten Mikrostrukturierungstechniken in das Aufnahmeelement 40 mit einer Tiefe von z. B. 0.01 µm bis 100 µm gefräst oder geätzt, so dass für adhärente Zellen ein wirksamer lateraler Schutz gebildet wird. Auf dem Boden der Vertiefung 46 oder allgemein auf der Oberfläche des Aufnahmeelements 40 (Figur 1H) kann eine adhäsionsfördernde Beschichtung 47 vorgesehen sein, die wenigstens einen Teilbereich, zum Beispiel die Mitte der Oberfläche 41 bedeckt. Die Beschichtung 47 besteht aus Fibronectin mit einer Dicke von 10 nm bis zu einigen µm. Sie wird von einem adhäsionsmindernden Rand 48 aus PTFE umgeben. Alternativ kann die Beschichtung physiologisch wirksame Moleküle zu Erzeugung von Wechselwirkungen mit den Zellen enthalten.

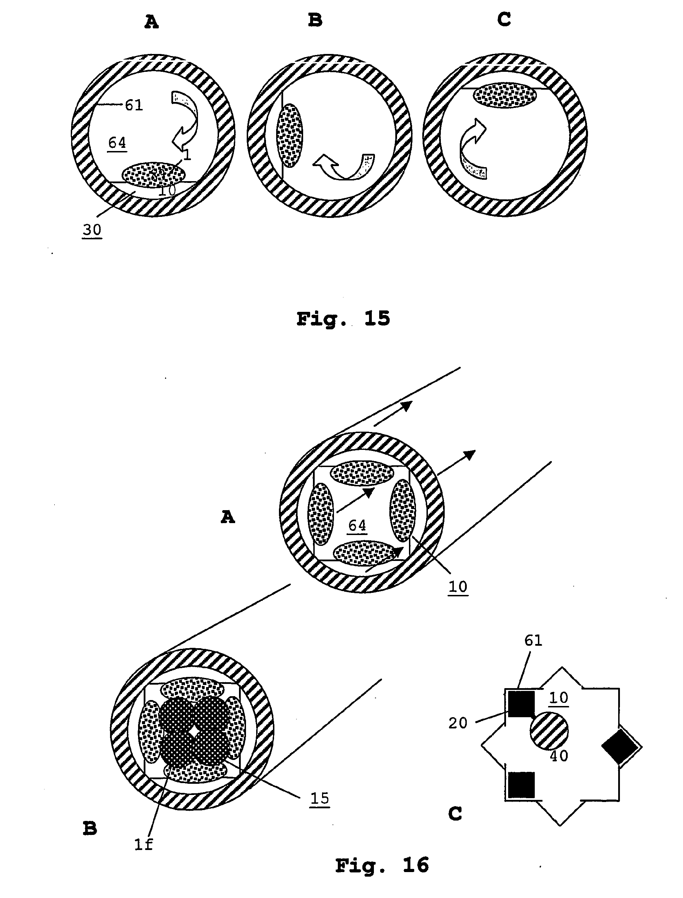

Die Figuren 1I und 1J illustrieren Zellträger mit Bodenelementen, die eine profilierte Auflage 33 mit einem konvex oder konkav gewölbte Auflageprofil bilden. Diese Zellträger sind mit ihren zylindrischen Auflageprofilen für eine stabile Auflage auf gewölbten Basisflächen geeignet, die in den Figuren 15 und 16 gezeigt sind.

Figur 1K zeigt seitliche Verankerungselemente 12, die als Vorsprünge und Ausnehmungen mit relativ zueinander komplementären Formen an den Seiten des Zellträgers angebracht sind. Sie dienen der gegenseitigen Verankerung benachbarter Zellträger und ggf. bei genügender lateraler Ausdehnung (siehe gestrichelte Variante) als Stützelemente. Feste Zellträgerverbindungen, die ggf. nicht mehr gelöst werden können, können zum Beispiel an den Ausgängen eines Mikrokanalsystems von Vorteil sein, wenn eine Zellträgergruppe in ein neues System oder eine Kultivierungskammer überführt werden soll. Auf diese Weise lässt sich ein hoher Automatisierungsgrad erreichen, wie er für die Anwendungen in der Biotechnologie gewünscht ist.

Gemäß Figur 1L werden das Magnetelement 20 und das Bodenelement 30 als separate Teile bereitgestellt. Beispielsweise kann das Magnetelement 20 nach Art eines herkömmlichen Magnetbeads mit der Probe 1 verbunden sein, die auf der Oberfläche des Bodenelements 30 angeordnet ist.

Figur 1M illustriert eine Variante des Zellträgers mit einem sphärischen Aufnahmeelement 40, das sich durch eine vergrößerte Adsorptionsoberfläche auszeichnet.

Figur 2 zeigt weitere Ausführungsbeispiele des Zellträgers 10 in schematischer Perspektivansicht. Die Figuren 2A und 2B illustrieren wie die Figuren 1B und 1H Oberflächenbereiche 42 (strukturierte Felder), an denen Zellen 1 gut anhaften und überleben können. Auf der Oberfläche des Auflageelementes ist gemäß Figur 2A das Identifizierungselement 13 in Form eines Strichcodes angebracht.

Die Oberflächenbereiche 42, an denen Zellen 1 gut anhaften, können sich als streifenförmige Laufstrecken 42A zum Rand des Zellträgers erstrecken (siehe Figur 2B). Im zusammengesetzten Zustand als Kulturträger (siehe Figur 4) können die Laufstrecken 42A zur Zellwanderung zwischen benachbarten Zellträgern zusammenwirken.

Die Magnetelemente 20 des Zellträgers 10 sind kompakt (Figur 2A), als eingefügte Schicht (Figur 2B), als eingefügte Teilelemente (Figur 2C) oder als eine untere Beschichtung ausgeführt (Figur 2D). Wichtig ist, dass die Zellträger 10 durch das eigene Magnetfeld oder in einem äußeren Magnetfeld nicht dazu neigen, sich zu drehen oder zu kippen. Dadurch können Zellen nicht an Kanalwände oder eine Basisfläche (siehe unten) gedrückt werden und dort anhaften.

Die Geometrie der Zellträger 10 kann in weiten Bereichen frei gewählt werden, z.B. um ein Zusammenfügen oder eine Orientierungsbestimmung zu erleichtern. Die Figuren 2A bis 2C zeigen eine polygonale Außenform 14 mit einer Vorzugsrichtung (A, B) oder mit einer hexagonalen Symmetrie (C).

Gemäß weiteren, nicht dargestellten Varianten weist der Zellträger eine regelmäßig achteckige, rechteckige, quadratische, ellipsenförmige oder kreisförmige Grundfläche auf.

Figur 3A zeigt den Zellträger 10 in konus- oder pyramidenstumpfförmiger Ausführung mit der Vertiefung 46 im Zentralbereich, in der eine oder mehrere Zellen 1 angesiedelt werden können, die einen gewissen Schutz durch diese geometrische Ausformung erhalten. Das schichtförmige Magnetelement 20 ist gegenüber der Bodenseite durch das Bodenelement 30 überschichtet. Das dargestellte Ausführungsbeispiel verfügt über ausgezeichnete Lagestabilität bei gleichzeitiger Freiheit der Gestaltung der Umgebung der Zellkultivierungsfläche. Das Aufnahmeelement des Zellträgers 10 gemäß Figur 3 wird zum Beispiel durch anisotropes Ätzen aus Silizium hergestellt.

Figur 3B zeigt den Zellträger 10 mit einem Magnetelement, das Teilelemente 22, 23 umfasst. Der Zellträger 10 besteht aus einer transparenten Platte, z. B. aus Glas oder Kunststoff, auf deren Oberfläche als Aufnahmeelement 40 eine strukturierte, lichtundurchlässige Beschichtung 42 vorgesehen ist. In der Beschichtung 42 ist eine kreisförmige Ausnehmung 43 gebildet. Die Ausnehmung 43 stellt eine transparente Beobachtungsfläche dar, die eine optische Beobachtung einer Probe auf dem Aufnahmeelement 40 ermöglicht. Am Rand der Beschichtung 42 liegt der transparente Zellträger frei. Analog zu der in Figur 2B gezeigten Variante sind Laufstrecken 42A zur Zellbewegung auf benachbarte Zellträger vorgesehen.

Ausführungsbeispiele erfindungsgemäßer Kulturträger 50 jeweils mit mehreren Zellträgern 10 sind in Figur 4 gezeigt. Die Varianten A bis D illustrieren, wie aus Zellträgern 10 mit gleichen oder verschiedenen geometrischen Außenformen geordnete, musterartige Anordnungen realisiert werden. Da die Zellträger flächendeckende Muster bilden, besitzen die Kulturträger 50 geschlossene Kulturträgeroberflächen 51, die als Substrate für Zellkulturen oder Zellmonolagen mit einer hohen Anpassungsfähigkeit für die konkrete Anwendung verwendet werden. Vorteilhafterweise können durch die Flexibilität bei der Zusammenstellung der Zellträger Zellkulturen mit einer definierten Lagezuordnung von spezifischen, ggf. verschiedenen Zellen geschaffen werden geschaffen.

Die Variante gemäß Figur 4A mit regelmäßigen, sechseckigen Zellträgern weist den Vorteil auf, dass die Zellträger mit einer großen Fläche der einzelnen Zellträger flächendeckend zusammensetzbar sind. Die Variante gemäß Figur 4D mit pfeilförmigen Zellträgern weist den Vorteil auf, dass eine geometrische Vorzugsrichtung geschaffen wird, die von außen erkennbar oder optisch detektierbar ist. Die pfeilförmigen Zellträger haben die Form eines Streifens mit einer Spitze an einem (vorderen) Ende und einer Einkerbung am entgegengesetzten (hinteren) Ende. Die Spitze und die Einkerbung sind komplementär zueinander geformt. Die vorderen und hinteren Enden definieren die geometrische Vorzugsrichtung, die bevorzugt mit einer magnetischen Ausrichtung des Magnetelements oder seiner Teile zusammenfällt. Weitere Zellträger können an beiden Enden Spitzen oder Einkerbungen aufweisen.

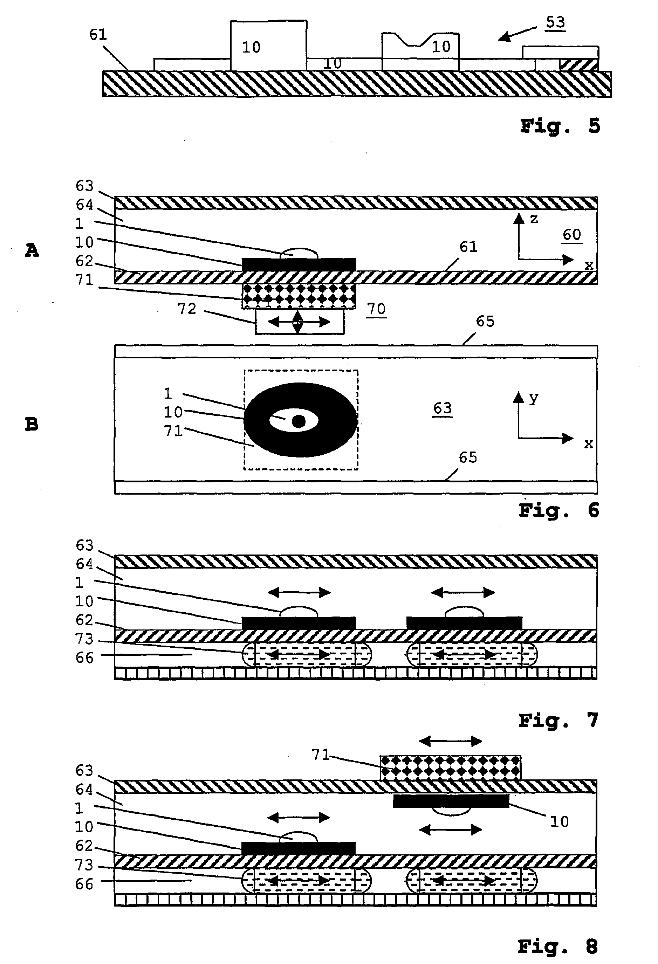

Ein weiterer Freiheitsgrad bei der Gestaltung der Zellkulturen auf erfindungsgemäßen Kulturträgern 50 ist in Figur 5 gezeigt. Wenn auf einer gemeinsamen Basisfläche 61 verschieden hohe Zellträger 10 kombiniert werden, entsteht eine Kulturträgeroberfläche 53 mit einer Stufentopographie. Dabei können die Zellträger aneinandergrenzend, sich übereinander schiebend oder miteinander verhakt oder verankert angeordnet sein.

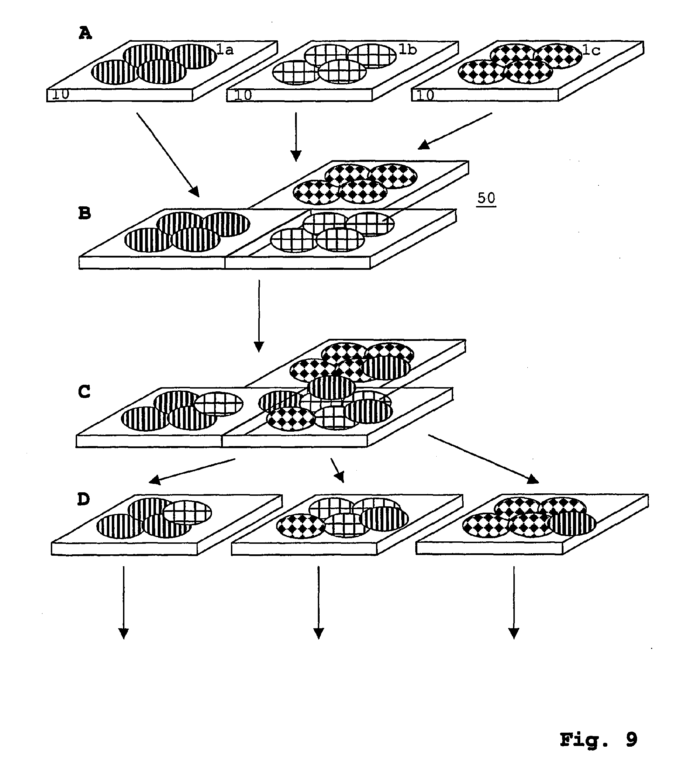

Einzelheiten von erfindungsgemäßen Manipulationseinrichtungen 60 für biologische Proben sind in den Figuren 6 bis 16 illustriert. Allgemein umfasst die Manipulationseinrichtung 60 eine feste Basisfläche 61, auf der mindestens ein Zellträger 10 positionierbar und beweglich ist, und eine Stelleinrichtung 70 zur Ausübung einer magnetischen Kraft auf den Zellträger 10. Diese Kombination ist erfindungsgemäß mit verschiedenen Einrichtungen zur Zellkultivierung, wie zum Beispiel mit Kulturschalen oder anderen Arbeitsplattformen realisierbar. Die folgende Beschreibung bezieht sich auf die bevorzugte Realisierung der Manipulationseinrichtung 60 mit einem fluidischen Mikrosystem, das sich durch eine miniaturisierte Ausführung von flüssigkeitsgefüllten Kompartimenten und Kanälen zur Aufnahme der zu manipulierenden Zellen auszeichnet. Fluidische Mikrosysteme und ihre Herstellung aus strukturierten Boden- oder Deckplatten, die durch Abstandshalter voneinander getrennt sind, zum Beispiel aus Kunststoff, Glas und/oder Halbleitermaterialien sind an sich bekannt, so dass hier deren Einzelheiten nicht beschrieben werden.

Im Folgenden wird auf horizontal ausgerichtete Mikrosysteme Bezug genommen. Die Erfindung ist entsprechend mit vertikal oder anders geneigt ausgerichteten Basisflächen umsetzbar, soweit durch eine inherente Magnetbindung, mit der Stelleinrichtung oder einen anderen Haftmechanismus dafür gesorgt wird, dass Zellträger sich im ruhenden Zustand nicht unter der Wirkung der Gravitation verschieben.

Figur 6A zeigt als Teil der Manipulationseinrichtung 60 in Schnittansicht einen Kanal 64, der zwischen einer Bodenplatte 62 und einer Deckplatte 63 gebildet ist. Die feste Basisfläche 61 wird durch die zum Kanal 64 weisende Oberfläche der Bodenplatte 62 gebildet. Die Boden- und Deckplatten 62, 63 sind transparent und z. B. aus Glas, Silizium und/oder Kunststoffmaterial mit einer Dicke von z. B. 50 µm bis 3 mm hergestellt. Der Abstand in z-Richtung zwischen den Boden- und Deckplatten 62, 63 beträgt zum Beispiel 10 µm bis 5 mm. Die Kanalbreite in y-Richtung beträgt zum Beispiel 40 µm bis 10 mm. Der Kanal 64 ist mit einer Flüssigkeit gefüllt, die ruhen oder strömen kann. Handelt es sich bei der Flüssigkeit um ein physiologisches Medium oder Nährmedium, so können Zellen auf dem Zellträger 10 am Leben erhalten, ggf. auch auf dem Zellträger wachsend zur Vermehrung gebracht werden. Die Oberflächen der Boden- und Deckplatten können zumindest in Teilbereichen mit PTFE oder einem ähnlich adhäsionsmindernden Material beschichtet sein. Ein Kanal besitzt beispielsweise die folgenden Dimensionen: Höhe kleiner als 1 mm, Breite kleiner als 10 mm, vorzugsweise kleiner als 5 mm, Länge: abhängig von der konkreten Anwendung, zum Beispiel cm- bis m-Bereich.

Erfindungsgemäß wird der Zellträger 10 mit mindestens einer Zelle 1 durch die Kanalwand (hier: Bodenplatte 62) hindurch von außen mit der Stelleinrichtung 70 in einem Magnetfeld gehalten. Die Bewuchsseite des Zellträgers 10 ist immer zum Kanal 64 hin gerichtet. Die Stelleinrichtung 70 weist mindestens ein Kraftelement 71 auf, das hier ein Permanentmagnet, ein magnetisierbarer Partikel oder eine Spule ist. Das Kraftelement 71 kann mit der Verschiebeeinrichtung 72 relativ zur Bodenplatte 62 mechanisch bewegt werden. Die Bewegung erfolgt je nach der konkreten Anwendung in mindestens einer Vortriebsrichtung, zum Beispiel parallel zur Längsrichtung des Kanals (x-Richtung), und allgemein in allen Raumrichtungen. Die Verschiebeeinrichtung 72 umfasst einen Stellmotor oder einen piezoelektrischen Antrieb. Durch die flache Ausführung und die geringe Größe (z.B. zur Bewegung anhaftender humaner Zellen oder Säugerzellen mit einem Durchmesser von 10 µm bis 100 µm) kann der Zellträger in definierter Position gehalten und auf der Innenseite des Kanals 64 ohne Verletzung der Sterilität mit einer Genauigkeit < 0.1 µm bewegt werden.

Figur 6B zeigt die Anordnung des Zellträgers 10 mit der Zelle 1 auf der Bodenplatte 62 zwischen Kanal-Seitenwänden 65 in der Draufsicht. Der Zellträger 10 besitzt eine ellipsenförmige Außenform, die von der Form des gestrichelt gezeigten Kraftelements 71 abweicht. In jedem Betriebszustand besitzt die Zelle 1 einen ausreichenden Abstand von den Wänden des Kanals 64.

Gemäß Figur 7 kann das Kraftelement durch Tropfen 73 einer magnetischen Flüssigkeit gebildet werden, die in einem weiteren Kanal 66 fluidisch getrennt vom ersten Kanal 64 bewegt werden. Die Bewegung der magnetischen Flüssigkeit erfolgt als kontinuierliche oder zeitweilig unterbrochene Flüssigkeitsströmung im Kanal 66. Die magnetische Flüssigkeit ist ein Öl, in dem magnetische oder magnetisierbare Partikel dispers verteilt sind. Die magnetischen Tropfen 73 sind durch Flüssigkeitsbereiche voneinander getrennt, mit denen die magnetische Flüssigkeit nicht mischbar ist. Auf diese Weise lassen sich Zellträger hintereinander in vorgebbaren, konstanten Abständen bewegen. Analog können Kraftelemente z. B. in Form von Permanentmagneten oder Spulen verwendet werden.

Die Bewegung von mehreren Zellträgern 10 kann an verschiedenen Kanalwänden erfolgen, wie in Figur 8 gezeigt ist. Ein erster Zellträger 10 wird mit dem Kraftelement 73 einer ersten Stelleinrichtung an der Bodenplatte 62 bewegt, während unabhängig davon ein zweiter Zellträger 10 wird mit dem Kraftelement 71 einer zweiten Stelleinrichtung an der Deckplatte 63 bewegt wird. Die Stelleinrichtungen können gleich oder wie dargestellt verschieden aufgebaut sein.

Die Figuren 9 und 10 illustrieren Ausführungsbeispiele erfindungsgemäßer Verfahren, bei denen allgemein Zellträger 10 zueinander benachbart positioniert werden, so dass Zellen und/oder Zellbestandteile auf den Zellträgern miteinander in Wechselwirkung treten können. Dabei ist z. B. eine Zellbewegung von einem auf einen benachbarten Zellträger (Figur 9) und/oder eine adhärente Wechselwirkung zwischen Zellen auf verschiedenen Zellträgern (Figur 10) vorgesehen.

Zur Handhabung verschiedener Zelltypen 1a, 1b und 1c werden diese zunächst auf getrennten Zellträgern 10 bereitgestellt (Figur 9, Schritt A). Das Beladen eines Zellträgers mit einer Zelle erfolgt zum Beispiel durch einen Sedimentationsvorgang in einem Kulturmedium, mit einer optischen Pinzette oder mit einer dielektrophoretischen Manipulation. Auf die oben beschriebene Weise lassen sich die Zellträger 10 z. B. in einem Kanalsystem oder einem anderen Flüssigkeitsvolumen zu einem Kulturträger 50 zusammenfügen (Figur 9, Schritt B). Nun können die Zellen durch die ihnen eigene aktive Bewegung oder über Filopodien den Kontakt zueinander suchen und Aggregate bilden, die erwünscht sind (Figur 9, Schritt C). Danach können die entstandenen Gruppierungen bei Bedarf auch wieder getrennt werden (Figur 9, Schritt D), beispielsweise um einer weiteren Kultivierung unterzogen zu werden.

Entsprechend der in Figur 9 gezeigten Verfahrensweise können zum Beispiel adulte Stammzellen tierischer oder humaner Herkunft, Blutzellen, Krebszellen oder Immunzellen miteinander zur Wechselwirkung gebracht werden.

Der in Figur 9 illustrierte Ablauf besitzt den Vorteil, dass schonend und ohne eine unphysiologische Beanspruchung der Zellen diese im adhärenten Zustand in vorbestimmter Weise zur Wechselwirkung gebracht werden können.

In Figur 10 sind in drei Schritten A, B und C wesentliche Phasen einer Kontaktaufnahme und Verbindung zwischen verschiedenen Zellen 1d, 1e auf verschiedenen Zellträgern 10 gezeigt. Der Zell-Zell-Kontakt spielt für das Tissue Engineering und besonders auch für Stammzelltherapien in der Medizin bei der "Prägung" von Zellen mit dem Ziel z. B. einer Differenzierung in einen bestimmten Zelltyp eine fundamentale Rolle. Analog zu Figur 8 lassen sich an den Boden- und Deckplatten 62, 63 eines Kanals oder eines flächenhaften Kompartiments Zellträger 10 unabhängig bewegen, auf denen sich die Zellen 1d, 1e befinden, die in Kontakt gebracht werden sollen (Schritt A). Mit magnetischen Kräften werden die Zellen übereinander platziert, so dass ein Spalt zwischen ihnen von weniger als 10 µm verbleibt (Schritt B).

Die meisten tierischen und humanen Zellen beginnen dann, selbständig Filopodien 2 auszustrecken und den Kontakt zueinander aktiv zu suchen (bei Schritt B durch schwarze Striche zwischen den Zellen dargestellt). Die lokalen Kontakte werden nachfolgend aktiv ausgebaut, bis ein intensiver und großflächiger Zellkontakt realisiert ist (Schritt C). Im weiteren Verfahren kann dann der Zellträgerverbund 15 aus zwei mit den Aufnahmeelementen zueinanderweisenden Zellträgern, die über die adhärenten Zellen verbunden sind, einer weiteren Bewegung und/oder Manipulation insbesondere entsprechend der erfindungsgemäßen Technik unterzogen werden.

Die Figuren 11 und 12 illustrieren weitere Einzelheiten der Kraftelemente von magnetischen Stelleinrichtungen. Unter der Halteplatte 67, auf der sich ein Zellträger 10 mit einer Zelle befindet, sind in Figur 11 verschieden geformten Magneten 74, 75 und 76 illustriert, die zur Bewegung von Zellträgern von außen an die Halteplatte 67 herangeführt und dann lateral verschoben werden. Zur Ausformung möglichst lokaler Magnetfelder sind kleine Anker (74), Spitzen (75) und/oder Permanentmagneten (76) einsetzbar. Die zur Halteplatte 67 weisende Seite des Magneten kann deutlich kleiner als der Zellträger 10 sein. Durch geeignete Wahl von Permanentmagneten lassen sich auch Abstoßungskräfte aufbauen, wenn sich Zellträger zu nahe kommen sollten (76).

Gemäß einer abgewandelten Variante (nicht dargestellt) umfasst ein Kraftelement einen zylindrischen Aufbau mit einem permanent-magnetischen Kern (Durchmesser rd. 2 mm) und einem äußeren Gehäuse (Durchmesser rd. 8 mm) zum Schutz des Kerns, zur Halterung des Kraftelements in der Stelleinrichtung und zur ggf. Feldformung. Der permanent-magnetische Kern ist beispielsweise in Kunststoff, z. B. PVC, im Gehäuse, z. B. aus Eisen oder Stahl, eingebettet. Anstelle des permanent-magnetischen Kerns kann eine elektrisch anregbare Spule vorgesehen sein.

Figur 12 illustriert schematisch einen Vorrichtungsaufbau für ein komplexes Multikanalsystem. Auf der Bodenplatte 62 eines Mikrosystems befinden sich mehrere Zellträger 10 gemäß Figur 1E (Zellen nicht dargestellt), die unabhängig voneinander bewegt werden können. Bei Ausführung stabförmiger Kraftelemente 77 lassen sich die Zellträger 10 in der Ebene der Bodenplatte 62 umeinander und an jede gewünschte Position führen. Die stabförmigen Kraftelemente 77 bestehen zum Beispiel aus einem Trägerstab, an dessen freien Ende ein Permanentmagnet oder ein magnetisierbarer Partikel angebracht ist. Zur Bewegung der stabförmigen Kraftelemente 77 werden an sich bekannte x-y-z-Verfahreinheiten mit einer Genauigkeit im µm-Bereich verwendet. Unter der Bodenplatte 62 können sich je nach Bedarf eine Vielzahl parallel betätigbarer Kraftelemente 77 befinden. Auch wenn die Dimensionen der Kanäle im Bereich von Mikrometern bis zu Millimetern liegen, sind Gesamtsysteme von beachtlicher flächenhafter Ausdehnung realisierbar. Das kann bis zu quadratmetergroßen Platten aus Glas, Plastik, Keramik, Silizium etc. reichen.

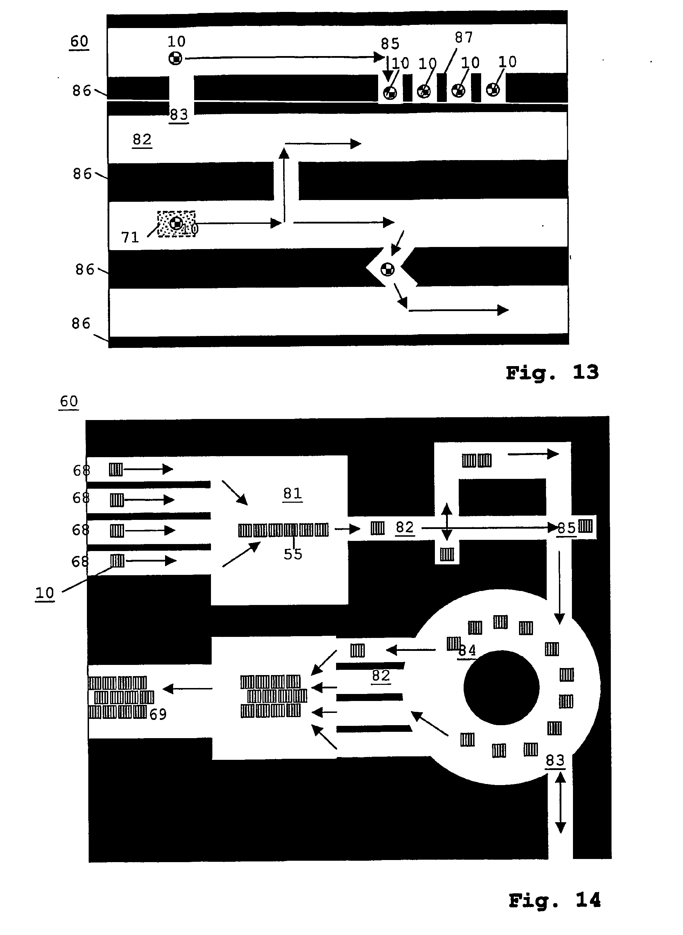

Die Basisfläche 61 einer erfindungsgemäßen Manipulationseinrichtung 60 besitzt vorzugsweise eine Oberflächenstruktur 80 mit Vorsprüngen und/oder Vertiefungen, mit der verschiedene Bereiche mit spezifischen Funktionen voneinander abgegrenzt werden können. Bei fluidischen Mikrosystemen wird die Oberflächenstruktur 80 durch die Trennwände z. B. von Kanälen oder flächigen Kompartimenten gebildet. Die Variabilität bei der Gestaltung von Mikrosystemen, deren Oberflächenstruktur Freiflächen 81, Kanäle 82, Verzweigungen 83, Ringbahnen 84 und Höfe 85 umfasst, ist beispielhaft in den Figuren 13 und 14 gezeigt.

Figur 13 illustriert ein Vierkanalsystem in Draufsicht, wobei helle Bereiche flüssigkeitsgefüllte Kanäle 82 zeigen, während schwarze Bereiche Kanalwände 86 und Spacer-Bereiche 87 zeigen. In den hellen Bereiche ist die Basisfläche für die Zellträger transparent. Runde Zellträger 10 (hier ohne Zellen gezeigt) lassen sich auf der Bodenplatte 62 mittels eines magnetischen oder magnetisierbaren Kraftelementes 71 entlang der Pfeile bewegen.

Wichtige Grundelemente, die in Mikrosystemen für die Kombination und den Zellbewuchs (von der Einzelzelle bis zur Zellgruppe) bevorzugt verwendet werden, umfassen Verzweigungen 83 mit Durchgängen zwischen den Kanälen 82 und Höfe 85, die als Parkpositionen für Zellträger verwendbar sind (Figur 13). Parallel verlaufende Kanäle können auch als Überholstrecken verwendet werden. Die Kombination derartiger Elemente erlaubt das Design komplexer Mikrokanalsysteme für die Zellmanipulation und *in vitro* Kultivierung unter absoluter Sterilität, mit vielfältigen Möglichkeiten z. B. der Beobachtung und Beleuchtung.

Die Höfe 85 bilden geschützte Ruhepositionen für die Zellträger, an denen bestimmte Verfahrensschritte, wie zum Beispiel ein Kulturwachstum, die Wechselwirkung mit einem Wirkstoff und/oder die Ausbildung oder Festigung der Bindung der Zelle mit dem Zellträger ausgeführt werden können.

Erfindungsgemäße Manipulationseinrichtungen (insbesondere Mikrokanalsysteme) können des Weiteren mit optischen, elektrischen und fluidischen Komponenten ausgestattet sein. Es können zum Beispiel Elektroden zur Erzeugung elektrischer Felder (Elektroporation, Elektrofusion, Elektroosmose, Dielektrophorese, Elektrophorese) vorgesehen sein.

In Figur 14 ist ein Verfahren illustriert, bei dem zunächst an parallelen Eingängen 68 der Manipulationseinrichtung 60 Zellträger 10 mit bestimmten Zellen eingesetzt werden. Die Freifläche 81 bildet einen Gruppierungsbereich zur Formung einer Zellträgerkette 55, deren einzelne Zellträger im Kanal 82 zur Durchführung spezifischer Wechselwirkungen in Höfe 85, einen Kreuzkanal oder einen Nachbarkanal, der eine Parkschleife bildet, überführt werden. Des Weiteren können die Zellträger auf einer Ringbahn 84 angeordnet werden, die einen Zellträger-Kombinations- oder Gruppierungsring bildet. Von der Ringbahn 84 kann eine Verzweigung 83 in einen weiteren Kanal 82 oder hin zu Ausgängen 69 führen.'

Figur 14 illustriert die universellen Möglichkeiten der Zellträgermanipulation und -gruppierung verdeutlichen, wie sie für das Tissue Engineering und die "Prägung" von Zellen über deren Oberflächen in der Biotechnologie benötigt werden. Analog lassen sich noch weitaus komplexere Kanalsysteme entwickeln und miteinander vernetzen und kombinieren.

Die Umsetzung der Erfindung ist nicht auf rechteckige Strukturen beschränkt. Figur 15 zeigt einen Zellträger 10 gemäß Figur 1I auf einer gekrümmten Basisfläche 61, die durch die Innenwand eines Hohlzylinders, zum Beispiel einer Kapillare gebildet wird. Das Innenvolumen des Hohlzylinders bildet einen flüssigkeitsdurchströmten Kanal 64. Bei geeigneter Ausformung des Bodenelements 30 der Zellträger 10 können diese mit Zellen 1 in gewünschter Weise auf alle Seiten bewegt werden (Teilbilder B und C). Daraus ergeben sich sehr viele Möglichkeiten, eine bestimmte Nachbarschaft für Zellen bereitzustellen. Insbesondere wird die Aufgabe der definierten Gruppierung verschiedener oder gleichartiger Zelltypen gelöst, was insbesondere beim Tissue Engineering angewendet wird.

In Figur 16 sind beispielhaft weitere Grundkonfigurationen zur Zellaggregation dargestellt. In einem zylindrischen Kanal 64 befinden sich vier Zellträgern 10, auf denen sich gleichartige oder verschiedene Zellen 1 befinden. Diese können seitlich zueinander Kontakt aufnehmen (16 A) oder auch durch Vermehrung den verbleibenden Innenraum füllen, so dass auch dort Zellen 1f anzutreffen sind (16B). Dadurch entsteht aus den vier Zellträgern ein zusammenhängender Zellträgerverbund 15, der wiederum als Einheit weiter manipuliert werden kann. Bei den Zellen im Innenteil kann es sich auch um über die Flüssigkeit eingespülte Zellen oder synthetische Körper handeln, so dass sehr weitreichende und variable Formen der definierten Bildung von Aggregaten für die Biotechnologie verfügbar werden.

An dieser Stelle sei angemerkt, dass die magnetischen Kräfte groß genug gemacht werden können, um ein Zellaggregat auch wieder zu trennen. Diese Aufgabe ist mit den meisten anderen Verfahren (Dielektrophorese, optische Pinzetten etc.) nicht umsetzbar, jedoch eine Grundforderung des Tissue Engineering. Dieser Prozess kann schnell erfolgen, wobei mit Zellschädigungen zu rechnen ist, er kann aber auch mit einer Geschwindigkeit ausgeführt werden, die der Fluktuationsrate der Adhäsionskontakte der Zellen entspricht. Das wären dann Bewegungsgeschwindigkeiten im Bereich von wenigen bis zu einigen 100 µm/h. Derartige Geschwindigkeiten lassen sich leicht über die externen Magnetfelder realisieren.

Figur 16C zeigt eine eckige Kanalführung im Querschnitt, die eine eckige Basisfläche 61 bildet. Zellträger 10 mit magnetisierbaren oder magnetischen Magnetelementen 20 (schwarz) können in Bahnen, Nuten etc. bewegt werden. An ihnen können sich als Aufnahmeelemente 40 z.B. sphärisch ausgeformte Teile befinden, an denen nun wieder die Zellen (hier nicht dargestellt) anhaften können. Auf diese Weise wird ein gewisser Abstand der Zellen zur Kanaloberfläche erreicht, was hilft, die Anhaftung der Zellen an der Kanalwand zu verhindern. Analog können Schienen, Führungen und Verdickungen/Verdünnungen in der Kanalform ausgeführt werden.

## Patentansprüche

1. Zellträqer (10) zur Aufnahme mindestens einer biologischen Zelle, der ein Magnetelement (20), ein Bodenelement (30) und ein Aufnahmeelement (40) aufweist, wobei das Aufnahmeelement (40) an einer zum Bodenelement (30) gegenüberliegenden Seite des Zellträgers (10) angeordnet ist und eine Oberfläche (41) zur Aufnahme der biologischen Zelle aufweist,
**dadurch gekennzeichnet dass**
- der Zellträger (10) auf einer festen Basisfläche beweglich ist,
- das Bodenelement (30) eine stabile, auf der Basisfläche in mindestens einer Richtung verschiebbare Auflage (31) bildet, und
- das Aufnahmeelement (40) in einem vorbestimmten Teilbereich der Oberfläche (41) adhäsionsfördernd gebildet ist.

2. Zellträger nach Anspruch 1, bei dem das Bodenelement (30) eine ebene Auflage (31) bildet.

3. Zellträger nach Anspruch 2, bei dem die ebene Auflage (31) flächig oder durch mindestens drei Auflagepunkte (32) gebildet ist.

4. Zellträger nach Anspruch 1, bei dem das Bodenelement (30) eine profilierte Auflage (33) mit einem vorbestimmten Auflageprofil bildet.

5. Zellträger nach Anspruch 4, bei dem das Auflageprofil einen Ausschnitt einer Zylinderoberfläche bildet.

6. Zellträger nach mindestens einem der vorhergehenden Ansprüche, bei dem das Bodenelement (30) eine adhäsionsmindernde Beschichtung aufweist.

7. Zellträger nach mindestens einem der vorhergehenden Ansprüche, bei das Aufnahmeelement (40) wenigstens in einem Teilbereich wenigstens eine der Oberflächenformen besitzt, die eine ebene unstrukturierte Form (41), eine ebene strukturierte Form (42), eine Vertiefung (45) und einen Hohlraum (44) umfassen.

8. Zellträger nach mindestens einem der vorhergehenden An sprüche, bei dem das Bodenelement (30) und das Aufnahmeelement (40) übereinander angeordnet sind und einen schichtförmigen Aufbau bilden.

9. Zellträger nach Anspruch 8., der eine Dicke kleiner als 200 µm und eine Querschnittsfläche mit einer lateralen Dimension kleiner als 2 mm aufweist.

10. Zellträger nach mindestens einem der Ansprüche 7 bis 9, bei dem das Aufnahmeelement (40) einen mit einer adhäsionsmindernden Beschichtung versehenen Rand (47) aufweist.

11. Zellträger nach mindestens einem der vorhergehenden Ansprüche, der einen transparenten Teilbereich (43) aufweist.

12. Zellträger nach mindestens einem der vorhergehenden Ansprüche, bei dem das Magnetelement (20) das Bodenelement (30) und/oder das Aufnahmeelement (40) bildet oder einen separaten, mit der Probe (1) verbundenen Partikel umfasst.

13. Zellträger nach mindestens einem der vorhergehenden Ansprüche, bei dem das Magnetelement (20) aus wenigstens einem Material gebildet ist, das aus der Gruppe gewählt ist, die paramagnetische, ferromagnetische und diamagnetische Materialien umfasst.

14. Zellträger nach mindestens einem der vorhergehenden Ansprüche, bei dem das Magnetelement (20) aus wenigstens einer Induktionseinrichtung gebildet ist.

15. Zellträger nach mindestens einem der vorhergehenden Ansprüche, bei dem das Magnetelement (20) eine durch den Zellträger (10) verlaufende, durchgehende Schicht bildet.

16. Zellträger nach mindestens einem der vorhergehenden Ansprüche 1 bis 12, bei dem das Magnetelement (20) mehrere, im Zellträger (10) verteilte Teilelemente (22, 23) umfasst.

17. Zellträger nach Anspruch 16, bei dem die Teilelemente (22) mit einem Abstand von mindestens einem seitlichen Rand des Zellträgers (10) angeordnet sind.

18. Zellträger nach Anspruch 16, bei dem die Teilelemente (23) an mindestens einem seitlichen Rand des Zellträgers (10) angeordnet sind.

19. Zellträger nach mindestens einem der vorhergehenden Ansprüche, bei dem das Magnetelement (20) mindestens eine Speicherzelle (24) bildet.

20. Zellträger nach mindestens einem der vorhergehenden Ansprüche, der eine Außenform (12) besitzt, die auf verschiedenen Seiten zueinander komplementär geformte Teilstücke aufweist.

21. Zellträger nach mindestens einem der vorhergehenden Ansprüche, der eine polygonale oder eine runde Außenform (14) besitzt.

22. Zellträger nach mindestens einem der vorhergehenden Ansprüche, dessen Außenform (14) so gewählt ist, dass eine Vielzahl von Zellträgern (10) nebeneinander eine lückenlose Flächenpackung bilden.

23. Zellträger nach mindestens einem der vorhergehenden Ansprüche, der Verankerungselemente (12) aufweist, die mit mindestens einem Vorsprung und mindestens einer Ausnehmung seitlich am Zellträger (10) gebildet sind.

24. Zellträger nach mindestens einem der vorhergehenden Ansprüche, der mindestens ein Stützelement (11) aufweist, das seitlich vom Zellträger (10) absteht.

25. Zellträger nach Anspruch 24, bei dem mehrere Stützelemente (11) das Bodenelement bilden.

26. Zellträger nach mindestens einem der vorhergehenden Ansprüche, der mindestens ein Identifizierungselement (13) aufweist.

27. Zellträger nach mindestens einem der vorhergehenden Ansprüche, der zur Aufnahme mindestens einer biologischen Zelle (1) eingerichtet ist.

28. Zellträger nach mindestens einem der vorhergehenden Ansprüche, dessen Oberfläche (41) eine laterale Dimension aufweist, die im Bereich von 10 µm bis 1 cm gewählt ist.

29. Zellträger nach mindestens einem der vorhergehenden Ansprüche, der eine Höhe besitzt, die im Bereich von 0.5 µm bis 2000 µm gewählt ist.

30. Kulturträger (50) zur Aufnahme biologischer Zellen, der eine Vielzahl von Zellträgern (10) nach mindestens einem der vorhergehenden Ansprüche aufweist.

31. Kulturträger nach Anspruch 30, bei dem die Zellträger (10) nebeneinander angeordnet sind, wobei die Zellträger (10) sich gegenseitig berühren.

32. Kulturträger nach Anspruch 31, bei dem die Zellträger (10) miteinander verankert sind.

33. Kulturträger nach mindestens einem der Ansprüche 30 bis 32, bei dem die Aufnahmeelemente (40) der Zellträger (10) eine ebene Kulturträgeroberfläche (51) bilden.

34. Kulturträger nach mindestens einem der Ansprüche 30 bis 33, bei dem die Aufnahmeelemente (40) der Zellträger (10) eine gekrümmte Kulturträgeroberfläche (52) bilden.

35. Kulturträger nach mindestens einem der Ansprüche 30 bis 34, bei dem die Aufnahmeelemente (40) der Zellträger (10) eine Kulturträgeroberfläche (53) mit Stufen bilden.

36. Manipulationseinrichtung (60) für biologische Proben, die aufweist:
- eine feste Basisfläche (61), die zur Positionierung mindestens eines Zellträgers (10) nach mindestens einem der Ansprüche 1 bis 29 eingerichtet ist, und
- eine Stelleinrichtung (70) zur Ausübung einer magnetischen Kraft auf den mindestens einen Zellträger (10).

37. Manipulationseinrichtung nach Anspruch 36, bei der die Basisfläche (61) eine ebene Form besitzt.

38. Manipulationseinrichtung nach Anspruch 36, bei der die Basisfläche (61) eine gewölbte Form besitzt.

39. Manipulationseinrichtung nach mindestens einem der Ansprüche 36 bis 38, bei der die Basisfläche (61) eine Oberflächenstruktur (80) mit Vorsprüngen und/oder Vertiefungen besitzt.

40. Manipulationseinrichtung nach Anspruch 39, bei der die Oberflächenstruktur (80) mindestens ein Formelement bildet, das aus der Gruppe von Formelementen ausgewählt ist, die Freiflächen (81), Kanäle (82), Verzweigungen (83), Ringbahnen (84) und Höfe (85) umfasst.

41. Manipulationseinrichtung nach mindestens einem der Ansprüche 36 bis 40, bei der zwei Basisflächen (61) vorgesehen sind, deren freie Oberflächen zueinander weisend mit einem Abstand angeordnet sind und die beide zur Aufnahme von Zellträgern eingerichtet sind.

42. Manipulationseinrichtung nach mindestens einem der Ansprüche 36 bis 41, bei der die Basisfläche (61) zumindest in Teilbereichen ein magnetisches oder magnetisierbares Material aufweist.

43. Manipulationseinrichtung nach mindestens einem der Ansprüche 36 bis 42, bei der die Stelleinrichtung (70) mindestens ein Kraftelement (71, 73-77) aufweist, das aus der Gruppe der Kraftelemente ausgewählt ist, die Permanentmagneten, Paramagneten, Ferromagneten und Diamagneten, elektrisch anregbare Magneten und magnetische Flüssigkeiten umfasst.

44. Manipulationseinrichtung nach Anspruch 43, bei der das Kraftelement (71, 73-77) mit einer Verschiebeeinrichtung (72) relativ zur Basisfläche (61) verschiebbar ist.

45. Manipulationseinrichtung nach mindestens einem der Ansprüche 36 bis 44, die eine Monitoreinrichtung aufweist.

46. Verfahren zur Manipulation biologischer Zellen, mit den Schritten:
- Positionierung mindestens einer biologischen Zelle (1) auf mindestens einem Zellträger (10), der verschiebbar auf einer festen Basisfläche (61) angeordnet ist, und
- Bewegung des Zellträgers (10) mit der mindestens einen biologischen Zelle (1) auf der Basisfläche (61) durch Ausübung einer magnetischen Kraft.

47. Verfahren nach Anspruch 46, bei dem mehrere Zellträger zueinander benachbart positioniert werden, so dass Zellen (1) und/oder Zellbestandteile auf den Zellträgern miteinander in Wechselwirkung treten.

48. Verfahren nach Anspruch 47, bei dem sich Zellen (1) zwischen benachbarten Zellträgern (10) bewegen.

49. Verfahren nach Anspruch 47, bei dem sich Zellen (1) und/oder Zellbestandteile auf benachbarten Zellträgern gegenseitig berühren.

50. Verfahren nach Anspruch 49, bei dem eine zellbiologische Prägung von Zellen (1) auf benachbarten Zellträgern (10) erfolgt.

51. Verfahren nach Anspruch 50, bei dem die magnetische Kraft auf die Probe (1) und/oder auf den Zellträger (10) wirkt.

## Claims

1. Cell carrier (10) for receiving at least one biological cell, which has a magnet element (20), a base element (30) and a receiving element (40), the receiving element (40) being disposed on one side of the cell carrier (10) which is situated opposite the base element (30) and having a surface (41) for receiving the biological cell,
**characterised in that**
- the cell carrier (10) is moveable on a solid base,
- the base element (30) forms a stable support (31) which is displaceable on the base in at least one direction, and
- the receiving element (40) is formed in a predetermined partial region of the surface (41) to be adhesion-promoting.

2. Cell carrier according to claim 1, in which the base element (30) forms a level support (31).

3. Cell carrier according to claim 2, in which the level support (31) is formed to be flat or by means of at least three support points (32).

4. Cell carrier according to claim 1, in which the base element (30) forms a profiled support (33) with a predetermined support profile.

5. Cell carrier according to claim 4, in which the support profile forms a cut-out of a cylinder surface.

6. Cell carrier according to at least one of the preceding claims, in which the base element (30) has an adhesion-reducing coating.

7. Cell carrier according to at least one of the preceding claims, in which the receiving element (40), at least in one partial region, has at least one of the surface forms which include a level unstructured form (41), a level structured form (42), a depression (45) and a cavity (44).

8. Cell carrier according to at least one of the preceding claims, in which the base element (30) and the receiving element (40) are disposed one above the other and form a laminar construction.

9. Cell carrier according to claim 8, which has a thickness of less than 200 µm and a cross-sectional area with a lateral dimension of less than 2 mm.

10. Cell carrier according to at least one of the claims 7 to 9, in which the receiving element (40) has an edge (47) which is provided with an adhesion-reducing coating.

11. Cell carrier according to at least one of the preceding claims, which has a transparent partial region (43).

12. Cell carrier according to at least one of the preceding claims, in which the magnet element (20) forms the base element (30) and/or the receiving element (40) or comprises a separate particle which is connected to the sample (1).

13. Cell carrier according to at least one of the preceding claims, in which the magnet element (20) is formed from at least one material which is chosen from the group which comprises paramagnetic, ferromagnetic and diamagnetic materials.

14. Cell carrier according to at least one of the preceding claims, in which the magnet element (20) is formed from at least one induction device.

15. Cell carrier according to at least one of the preceding claims, in which the magnet element (20) forms a continuous layer which extends through the cell carrier (10).

16. Cell carrier according to at least one of the preceding claims 1 to 12, in which the magnet element (20) comprises a plurality of partial elements (22, 23) which are distributed in the cell carrier (10).

17. Cell carrier according to claim 16, in which the partial elements (22) are disposed at a spacing from at least one lateral edge of the cell carrier (10).

18. Cell carrier according to claim 16, in which the partial elements (23) are disposed on at least one lateral edge of the cell carrier (10).

19. Cell carrier according to at least one of the preceding claims, in which the magnet element (20) forms at least one storage cell (24).

20. Cell carrier according to at least one of the preceding claims, which has an outer form (12) which has partial pieces which are formed on different sides in a complementary manner to each other.

21. Cell carrier according to at least one of the preceding claims, which has a polygonal or a round outer form (14).

22. Cell carrier according to at least one of the preceding claims, the outer form (14) of which is chosen such that a large number of cell carriers (10) form a gap-free flat packing next to each other.

23. Cell carrier according to at least one of the preceding claims, which has anchoring elements (12) which are formed with at least one projection and at least one recess laterally on the cell carrier (10).

24. Cell carrier according to at least one of the preceding claims, which has at least one support element (11) which protrudes laterally from the cell carrier (10).

25. Cell carrier according to claim 24, in which a plurality of support elements (11) forms the base element.

26. Cell carrier according to at least one of the preceding claims, which has at least one identification element (13).

27. Cell carrier according to at least one of the preceding claims, which is equipped to receive at least one biological cell (1).

28. Cell carrier according to at least one of the preceding claims, the surface (41) of which has a lateral dimension which is chosen in the range of 10 µm to 1 cm.

29. Cell carrier according to at least one of the preceding claims, which has a height which is chosen in the range of 0.5 µm to 2,000 µm.

30. Culture carrier (50) for receiving biological cells, which has a large number of cell carriers (10) according to at least one of the preceding claims.

31. Culture carrier according to claim 30, in which the cell carriers (10) are disposed next to each other, the cell carriers (10) touching each other mutually.

32. Culture carrier according to claim 31, in which the cell carriers (10) are anchored to each other.

33. Culture carrier according to at least one of the claims 30 to 32, in which the receiving elements (40) of the cell carriers (10) form a level culture carrier surface (51).

34. Culture carrier according to at least one of the claims 30 to 33, in which the receiving elements (40) of the cell carriers (10) form a curved culture carrier surface (52).

35. Culture carrier according to at least one of the claims 30 to 34, in which the receiving elements (40) of the cell carriers (10) form a culture carrier surface (53) with steps.

36. Manipulation device (60) for biological samples which has:
- a solid base (61) which is equipped to position at least one cell carrier (10) according to at least one of the claims 1 to 29, and
- an adjustment device (70) for exerting a magnetic force on the at least one cell carrier (10).

37. Manipulation device according to claim 36, in which the base (61) has a level form.

38. Manipulation device according to claim 36, in which the base (61) has a curved form.

39. Manipulation device according to at least one of the claims 36 to 38, in which the base (61) has a surface structure (80) with projections and/or depressions.

40. Manipulation device according to claim 39, in which the surface structure (80) forms at least one shaped element which is selected from the group of shaped elements which includes free surfaces (81), channels (82), branches (83), annular tracks (84) and crowns (85).

41. Manipulation device according to at least one of the claims 36 to 40, in which two bases (61) are provided, the free surfaces of which are disposed at a spacing and pointing towards each other and both are equipped to receive cell carriers.

42. Manipulation device according to at least one of the claims 36 to 41, in which the base (61) has a magnetic or magnetisable material at least in partial regions.

43. Manipulation device according to at least one of the claims 36 to 42, in which the adjustment device (70) has at least one force element (71, 73 - 77) which is selected from the group of force elements which comprises permanent magnets, paramagnets, ferromagnets and diamagnets, electrically excitable magnets and magnetic liquids.

44. Manipulation device according to claim 43, in which the force element (71, 73 - 77) is displaceable relative to the base (61), with a displacement device (72).

45. Manipulation device according to at least one of the claims 36 to 44, which has a monitor device.

46. Method for manipulation of biological cells, having the steps:
- positioning of at least one biological cell (1) on at least one cell carrier (10) which is disposed displaceably on a solid base (61), and
- movement of the cell carrier (10) with the at least one biological cell (1) on the base (61) by exerting a magnetic force.

47. Method according to claim 46, in which a plurality of cell carriers are positioned adjacent to each other so that cells (1) and/or cell components interact with each other on the cell carriers.

48. Method according to claim 47, in which cells (1) move between adjacent cell carriers (10).

49. Method according to claim 47, in which the cells (1) and/or cell components touch mutually on adjacent cell carriers.

50. Method according to claim 49, in which a cell-biological embossing of cells (1) on adjacent cell carriers (10) is effected.

51. Method according to claim 50, in which the magnetic force acts on the sample (1) and/or on the cell carrier (10).

## Revendications

1. Support de cellules (10) pour le positionnement d'au moins une cellule biologique qui comporte un élément magnétique (20), un élément de fond (30) et un élément de positionnement (40), dans lequel l'élément de positionnement (40) est disposé sur un côté du support de cellules (10) opposé à l'élément de fond (30) et comporte une surface (41) pour le positionnement de la cellule biologique,
**caractérisé en ce que**
- le support de cellules (10) est mobile sur une surface de base fixe,
- l'élément de fond (30) forme un appui stable (31) déplaçable dans au moins une direction sur la surface de base
et
- l'élément de positionnement (40) est formé dans une zone partielle prédéterminée de la surface (41) de manière à favoriser l'adhérence.

2. Support de cellules selon la revendication 1, dans lequel l'élément de fond (30) forme un appui plan (31).

3. Support de cellules selon la revendication 2, dans lequel l'appui plan (31) est planiforme ou est formé par au moins trois points d'appui (32).

4. Support de cellules selon la revendication 1, dans lequel l'élément de fond (30) forme un appui profilé (33) avec un profil d'appui prédéterminé.

5. Support de cellules selon la revendication 4, dans lequel le profil d'appui forme une encoche d'une surface cylindrique.

6. Support de cellules selon au moins l'une des revendications précédentes, dans lequel l'élément de fond (30) est muni d'un revêtement diminuant l'adhérence.

7. Support de cellules selon au moins l'une des revendications précédentes, dans lequel l'élément de positionnement (40) présente, dans au moins une zone partielle, au moins l'une des formes de surface qui comprennent une forme plane non structurée (41), une forme plane structurée (42), un évidement (45) et une cavité (44).

8. Support de cellules selon au moins l'une des revendications précédentes, dans lequel l'élément de fond (30) et l'élément de positionnement (40) sont placés l'un sur l'autre et forment une structure stratifiée.

9. Support de cellules selon la revendication 8 qui a une épaisseur inférieure à 200 µm et une aire de section avec une dimension latérale inférieure à 2 mm.

10. Support de cellules selon au moins l'une des revendications 7 à 9, dans lequel l'élément de positionnement (40) comporte un bord (47) muni d'un revêtement diminuant l'adhérence.

11. Support de cellules selon au moins l'une des revendications précédentes qui comporte une zone partielle transparente (43).

12. Support de cellules selon au moins l'une des revendications précédentes, dans lequel l'élément magnétique (20) forme l'élément de fond (30) et/ou l'élément de positionnement (40) ou comprend une particule séparée reliée à l'échantillon (1).

13. Support de cellules selon au moins l'une des revendications précédentes, dans lequel l'élément magnétique (20) est constitué d'au moins un matériau qui est choisi dans le groupe qui comprend les matériaux paramagnétiques, ferromagnétiques et diamagnétiques.

14. Support de cellules selon au moins l'une des revendications précédentes, dans lequel l'élément magnétique (20) est constitué par au moins un dispositif d'induction.

15. Support de cellules selon au moins l'une des revendications précédentes, dans lequel l'élément magnétique (20) forme une couche continue s'étendant à travers le support de cellules (10).

16. Support de cellules selon au moins l'une des revendications 1 à 12, dans lequel l'élément magnétique (20) comprend plusieurs éléments partiels (22, 23) répartis dans le support de cellules (10).

17. Support de cellules selon la revendication 16, dans lequel les éléments partiels (22) sont disposés à une distance d'au moins un bord latéral du support de cellules (10).

18. Support de cellules selon la revendication 16, dans lequel les éléments partiels (22) sont disposés sur au moins un bord latéral du support de cellules (10).

19. Support de cellules selon au moins l'une des revendications précédentes, dans lequel l'élément magnétique (20) forme au moins une cellule de stockage (24).

20. Support de cellules selon au moins l'une des revendications précédentes qui présente une forme extérieure (12) qui comporte des tronçons moulés de manière complémentaire sur différents côtés.

21. Support de cellules selon au moins l'une des revendications précédentes qui présente une forme extérieure (14) polygonale ou ronde.

22. Support de cellules selon au moins l'une des revendications précédentes dont la forme extérieure (14) est choisie de telle manière qu'une pluralité de supports de cellules (10) les uns à côté des autres forment un assemblage plan continu.

23. Support de cellules selon au moins l'une des revendications précédentes qui comporte des éléments d'ancrage (12) qui sont formés avec au moins une partie en saillie et au moins un évidement sur le côté du support de cellules (10).

24. Support de cellules selon au moins l'une des revendications précédentes qui comporte au moins un élément d'appui (11) qui part latéralement du support de cellules (10) .

25. Support de cellules selon la revendication 24, dans lequel plusieurs éléments d'appui (11) forment l'élément de fond.

26. Support de cellules selon au moins l'une des revendications précédentes qui comporte au moins un élément d'identification (13).

27. Support de cellules selon au moins l'une des revendications précédentes qui est agencé pour le positionnement d'au moins une cellule biologique (1).

28. Support de cellules selon au moins l'une des revendications précédentes dont la surface (41) présente une dimension latérale qui est choisie dans la plage de 10 µm à 1 cm.

29. Support de cellules selon au moins l'une des revendications précédentes qui a une hauteur qui est choisie dans la plage de 0,5 µm à 2000 µm.

30. Support de culture (50) pour le positionnement de cellules biologiques qui comporte une pluralité de supports de cellules (10) selon au moins l'une des revendications précédentes.

31. Support de culture selon la revendication 30, dans lequel les supports de cellules (10) sont disposés les uns à côté des autres, les supports de cellules (10) étant en contact les uns avec les autres.

32. Support de culture selon la revendication 31, dans lequel les supports de cellules (10) sont ancrés les uns avec les autres.

33. Support de culture selon au moins l'une des revendications 30 à 32, dans lequel les éléments de positionnement (40) des supports de cellules (10) forment une surface de support de culture plane (51).

34. Support de culture selon au moins l'une des revendications 30 à 33, dans lequel les éléments de positionnement (40) des supports de cellules (10) forment une surface de support de culture recourbée (52).

35. Support de culture selon au moins l'une des revendications 30 à 34, dans lequel les éléments de positionnement (40) des supports de cellules (10) forment une surface de support de culture (53) avec des gradins.

36. Dispositif de manipulation (60) pour des échantillons biologiques qui comprend :
- une surface de base fixe (61) qui est agencée pour le positionnement d'au moins un support de cellules (10) selon au moins l'une des revendications 1 à 29, et
- un dispositif de réglage (70) pour l'application d'une force magnétique sur le au moins un support de cellules (10).

37. Dispositif de manipulation selon la revendication 36, dans lequel la surface de base (61) a une forme plane.

38. Dispositif de manipulation selon la revendication 36, dans lequel la surface de base (61) a une forme bombée.

39. Dispositif de manipulation selon au moins l'une des revendications 36 à 38, dans lequel la surface de base (61) présente une structure superficielle (80) avec des parties en saillie et/ou des évidements.

40. Dispositif de manipulation selon la revendication 39, dans lequel la structure superficielle (80) forme au moins un élément moulé qui est choisi dans le groupe des éléments moulés qui comprend des surfaces libres (81), des canaux (82), des embranchements (83), des passages annulaires (84) et des couronnes (85).

41. Dispositif de manipulation selon au moins l'une des revendications 36 à 40, dans lequel il est prévu deux surfaces de base (61) dont les surfaces libres sont dirigées l'une vers l'autre à une certaine distance et qui sont toutes les deux agencées pour le positionnement de supports de cellules.

42. Dispositif de manipulation selon au moins l'une des revendications 36 à 41, dans lequel la surface de base (61) comporte un matériau magnétique ou magnétisable au moins dans des zones partielles.

43. Dispositif de manipulation selon au moins l'une des revendications 36 à 42, dans lequel le dispositif de réglage (70) comporte au moins un élément de force (71, 73-77) qui est choisi dans le groupe des éléments de force qui comprend les aimants permanents, les aimants ferromagnétiques et diamagnétiques, les aimants excitables électriquement et les liquides magnétiques.

44. Dispositif de manipulation selon la revendication 43, dans lequel l'élément de force (71, 73-77) peut être déplacé par rapport à la surface de base (61) à l'aide d'un dispositif de déplacement (72).

45. Dispositif de manipulation selon au moins l'une des revendications 36 à 44 qui comporte un dispositif d'écran de surveillance.

46. Procédé pour la manipulation de cellules biologiques avec les étapes de :
- positionnement d'au moins une cellule biologique (1) sur au moins un support de cellules (10) qui est placé de manière mobile sur une surface de base fixe (61), et
- déplacement du support de cellules (10) avec la au moins une cellule biologique (1) sur la surface de base (61) en exerçant une force magnétique.

47. Procédé selon la revendication 46, dans lequel plusieurs supports de cellules sont positionnés les uns à côté des autres de sorte que des cellules (1) et/ou des composants de cellules exercent une interaction réciproque sur les supports de cellules.

48. Procédé selon la revendication 47, dans lequel des cellules (1) se déplacent entre des supports de cellules adjacents (10).

49. Procédé selon la revendication 47, dans lequel des cellules (1) et/ou des composants de cellules sont en contact les uns avec les autres sur des supports de cellules adjacents.

50. Procédé selon la revendication 4.9, dans lequel on réalise une empreinte cytobiologique de cellules (1) sur des supports de cellules adjacents (10).

51. Procédé selon la revendication 50 dans lequel la force magnétique s'exerce sur l'échantillon (1) et/ou sur le support de cellules (10).
